# EUROPEAN PATENT APPLICATION

(11) **EP 2 595 208 A1**
(43) Date of publication of application: **22.05.2013**
(21) Application number: 11806612.5
(22) Date of filing: 24.06.2011
(51) Int. Cl.: H01L 51/50, C07D 209/86

(54) **LIGHT EMITTING ELEMENT**

(30) Priority: 13.07.2010 JP 2010158551
(71) Applicant: Toray Industries, Inc., Tokyo 103-8666 (JP)
(72) Inventor: TANAKA, Daisaku, Otsu-shi, Shiga 520-8558 (JP); MATSUKI, Shinichi, Otsu-shi, Shiga 520-8558 (JP); TOMINAGA, Tsuyoshi, Otsu-shi, Shiga 520-8558 (JP)
(74) Representative: Hager, Thomas Johannes
(86) International application number: PCT/JP2011/064512
(87) International publication number: WO 2012/008281

(57) **Abstract**

Provided is an organic thin film light emitting element which has achieved all of improved luminous efficiency, improved driving voltage and improved durability life. Specifically provided is a light emitting element which comprises a hole transport layer and an electron transport layer between a positive electrode and a negative electrode and emits light by means of electrical energy. The light emitting element is characterized in that: the hole transport layer of the light emitting element contains a compound represented by general formula (1); the electron transport layer contains a donor compound; and the donor compound is an alkali metal, an inorganic salt containing an alkali metal, a complex of an alkali metal and an organic substance, an alkaline earth metal, an inorganic salt containing an alkaline earth metal, or a complex of an alkaline earth metal and an organic substance. (In the formula, R¹-R²⁰ each represents one group selected from the group consisting of hydrogen, deuterium, an alkyl group, a cycloalkyl group, an amino group, an aryl group, a heterocyclic group, a heteroaryl group, an alkenyl group, a cycloalkenyl group, an alkynyl group, analkoxy group, an alkylthio group, an arylether group, an arylthioethergroup, a halogen, a cyano group, a -P(=O)R²⁴R²⁵ group and a silyl group;R²⁴ and R²⁵ each represents an aryl group or a heteroaryl group; and these substituents may be further substituted, or adjacent two substituents may combine together to form a ring. Meanwhile, R²¹-R²³ may be the same or different and each represents one group selected from the group consisting of an alkyl group, a cycloalkyl group, an aryl group and a heteroaryl group; and these substituents maybe further substituted.)

## Description

### TECHNICAL FIELD

The present invention is a light-emitting element that can convert electric energy into light, and relates to a light-emitting element that can be used in the fields of display elements, flat panel displays, backlights, illumination, interior materials, signs, signboards, digital cameras, light signal generators, etc.

### BACKGROUND ART

Research on organic thin-film light-emitting elements in which when the electrons injected from a cathode and the positive holes injected from an anode recombine in an organic fluorescent material, light is emitted is actively conducted in recent years. The light-emitting elements attract attention, since they are characterized by thin thickness, high-luminance light emission at low drive voltage and multi-color light emission obtained with selected fluorescent materials.

C. W. Tang et al. (Kodak) demonstrated that organic thin-film elements emit light at high luminance, and since then, studies have been conducted by many research organizations. In the typical configuration of the organic thin-film light-emitting elements presented by the research group of Kodak, a hole-transportable diamine compound, 8-hydroxyquinoline aluminum as a light-emitting layer, and Mg:Ag as a cathode are provided sequentially on an ITO glass substrate, and it can emit green light of 1,000 cd/m² at a drive voltage of approx. 10 V (for example, see non-patent document 1).

Thereafter, numerous examinations for practical application have been made, and as a result, organic thin-film light-emitting elements are employed, for example, in the main displays of cell phones, showing practical and steady growth. However, there still remain many technical problems, and above all, one of large problems is to obtain elements allowing a lower voltage and assuring a longer life.

The drive voltage of the element greatly depends on the carrier transport materials that transport carriers such as holes and electrons to the light-emitting layer. Of the materials, as the materials for transporting holes (hole transport materials), materials with a carbazole skeleton are known (patent documents 1 to 6).
Further, as techniques to lower the voltage of the element from the viewpoint of electron transport, techniques of doping an alkali metal into the material used as an electron transport layer are disclosed (see patent documents 7 to 11).

### PRIOR ART DOCUMENTS

### PATENT DOCUMENTS

Patent document 1: JP 8-3547 A
Patent document 2: JP 9-249876 A
Patent document 3: JP 11-144876 A
Patent document 4: JP 2008-294161 A
Patent document 5: JP 2003-133075 A
Patent document 6: Korean Patent Laid-open Gazette 2009-28943
Patent document 7: JP 2000-348864 A
Patent document 8: JP 2004-277377 A
Patent document 9: JP 2003-347060 A
Patent document 10: JP 2002-352961 A
Patent document 11: JP 2004-2297 A

### NON-PATENT DOCUMENT

Non-patent document 1: Applied Physics Letters (USA), 1987, vol. 51, no. 12, page 913

### OUTLINE OF THE INVENTION

### PROBLEMS TO BE SOLVED BY THE INVENTION

However, even if the materials of patent documents 1 to 6 are merely used, the light emission efficiency, drive voltage and durability life of the elements are insufficient. Further, the techniques of patent documents 7 to 11 are certainly effective for lowering the voltage of the elements, but still light emission efficiency and durability life are insufficient. Any technique for allowing both low voltage drive and assuring long durability life has not been found yet.

The object of this invention is to solve these problems of the prior art, and to provide an organic thin-film light-emitting element improved in all of light emission efficiency, drive voltage and durability life.

### MEANS FOR SOLVING THE PROBLEMS

A light-emitting element for emitting light by means of electric energy, in which at least a hole transport layer and an electron transport layer exist between an anode and a cathode, wherein the hole transport layer of the light-emitting element contains a compound represented by the following general formula (1), while the electron transport layer contains a donor compound, the donor compound being an alkali metal, an inorganic salt containing an alkali metal, a complex comprising an alkali metal and an organic substance, an alkaline earth metal, an inorganic salt containing an alkaline earth metal or a complex comprising an alkaline earth metal and an organic substance.

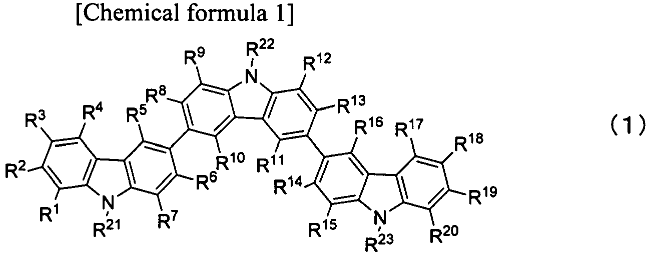

R¹ to R²⁰ each are selected from the group comprising hydrogen, deuterium, alkyl group, cycloalkyl group, amino group, aryl group, heterocyclic group, heteroaryl group, alkenyl group, cycloalkenyl group, alkynyl group, alkoxy group, alkylthio group, arylether group, aryl thioether group, halogen, cyano group, -P(=O)R²⁴R²⁵ and silyl group. These substituent groups may also be further substituted, or substituent groups adjacent to each other may also form a ring. R²⁴ and R²⁵ each denote an aryl group or heteroaryl group. These substituent groups may be further substituted, or substituent groups adjacent to each other may also form a ring. R²¹ to R²³ may be identical to or different from each other, and are selected from the group comprising an alkyl group, cycloalkyl group, aryl group and heteroaryl group. These substituent groups may also be further substituted.

### EFFECTS OF THE INVENTION

This invention can provide an organic electric-field light-emitting element driven at low voltage, having high light emission efficiency and further having sufficient durability life.

### BRIEF DESCRIPTION OF THE DRAWING

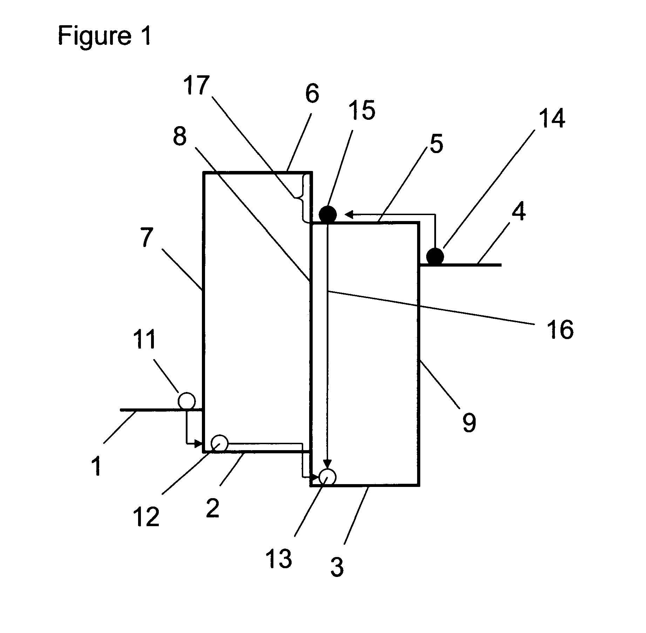

[Fig. 1] is a drawing showing the energy states of charges inside an organic electroluminescent element.

### MODES FOR CARRYING OUT THE INVENTION

The compounds represented by general formula (1) are explained below in detail. 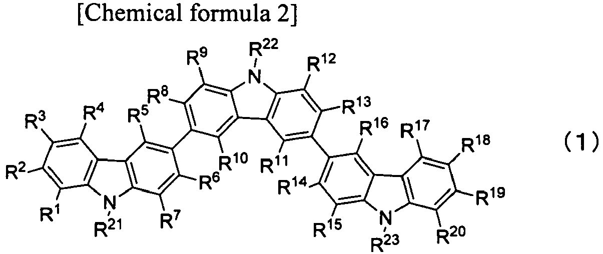 R¹ to R²⁰ each are selected from the group comprising hydrogen, deuterium, alkyl group, cycloalkyl group, amino group, aryl group, heterocyclic group, heteroaryl group, alkenyl group, cycloalkenyl group, alkynyl group, alkoxy group, alkylthio group, arylether group, aryl thioether group, halogen, cyano group, -P(=O)R²⁴R²⁵ and silyl group. These substituent groups may also be further substituted, or substituent groups adjacent to each other may also form a ring. R²⁴ and R²⁵ denote respectively an aryl group or heteroaryl group. These substituent groups may be further substituted, or substituent groups adjacent to each other may also form a ring. R²¹ to R²³ may be identical to or different from each other, and are selected from the group comprising an alkyl group, cycloalkyl group and heteroaryl group. These substituent groups may also be further substituted.

Among these substituent groups, the alkyl group denotes, for example, a saturated aliphatic hydrocarbon group such as a methyl group, ethyl group, n-propyl group, isopropyl group, n-butyl group, sec-butyl group or tert-butyl group, and the alkyl group may have a substituent group, though the substituent group is not necessarily required. In the case where the alkyl group is substituted, the additional substituent group is not especially limited, and can be, for example, an alkyl group or aryl group, etc. This applies also to the following. Further, the number of carbon atoms of the alkyl group is not especially limited but in view of raw material availability and cost, the number of carbon atoms is usually 1 to 20, and a more preferred range is 1 to 8. Furthermore, if the number of carbon atoms of the alkyl group is too large, the hole transportcapability may be inhibited and the heat resistance may decline. Consequently, a methyl group, ethyl group or t-butyl group is more preferred.

The cycloalkyl group denotes, for example, a saturated alicyclic hydrocarbon group such as a cyclopropyl group, cyclopentyl group, cyclohexyl group, norbornyl group or adamantyl group, and the cycloalkyl group may have a substituent group, though the substituent group is not necessarily required. The number of carbon atoms in the alkyl group portion is not especially limited, but is usually in a range from 3 to 20. If the number of carbon atoms is too large, the hole transportcapability may be inhibited. Consequently, a cyclopropyl group, cyclopentyl group or cyclohexyl group is more preferred.

The aryl group denotes, for example, an aromatic hydrocarbon group such as a phenyl group, naphthyl group, biphenyl group, phenanthryl group, fluorenyl group, anthracenyl group, pyrenyl group or terphenyl group. The aryl group may further have a substituent group, though the substituent group is not necessarily required. In the case where the aryl group is substituted, the additional substituent group is not especially limited, and can be, for example, an alkyl group, cycloalkyl group, heteroaryl group, alkoxy group or amino group, etc. The number of carbon atoms of the aryl group is not especially limited, but is usually in a range from 6 to 40.

The heterocyclic group denotes, for example, an aliphatic ring having an atom other than carbon in the ring such as pyran ring, piperidine ring or cyclic amide, and the heterocyclic group may have a substituent group, though the substituent group is not necessarily required. The number of carbon atoms of the heterocyclic ring is not especially limited, but is usually in a range from 2 to 20.

The heteroaryl group denotes a cyclic aromatic group having one atom or a plurality of atoms other than carbon in the ring such as a furanyl group, thiophenyl group, pyridyl group, quinolinyl group, pyrazinyl group, naphthyridyl group, benzofuranyl group, benzothiophenyl group, indolyl group, dibenzofuranyl group, dibenzothiophenyl group or carbazolyl group, and the heteroaryl group may be non-substituted or substituted. In the case where the heteroaryl group is substituted, the additional substituent group is not especially limited, and can be, for example, an alkyl group, cycloalkyl group, aryl group, alkoxy group or amino group, etc. The number of carbon atoms of the heteroaryl group is not especially limited, but is usually in a range from 2 to 30. From the viewpoints of making HOMO (Highest Occupied Molecular Orbital) level shallow and lowering the element drive voltage, dibenzofuranyl group, dibenzothiophenyl group or carbazolyl group is more preferred.

The alkenyl group denotes, for example, an unsaturated aliphatic hydrocarbon group containing a double bond such as a vinyl group, allyl group or butadienyl group, and the alkenyl group may have a substituent group, though the substituent group is not necessarily required. The number of carbon atoms of the alkenyl group is not especially limited but is usually in a range from 2 to 20.

The cycloalkenyl group denotes, for example, an unsaturated alicyclic hydrocarbon group containing a double bond such as a cyclopentenyl group, cyclopentadienyl group or cyclohexenyl group, and the cycloalkenyl group may have a substituent group, though the substituent group is not necessarily required.

The alkynyl group denotes, for example, an unsaturated aliphatic hydrocarbon group containing a triple bond such as an ethynyl group, and the alkynyl group may have a substituent group, though the substituent group is not necessarily required. The number of carbon atoms of the alkynyl group is not especially limited but is usually in a range from 2 to 20.

The alkoxy group denotes, for example, a functional group having an aliphatic hydrocarbon group bonded via an ether bond such as a methoxy group, ethoxy group or propoxy group, and the aliphatic hydrocarbon group may have a substituent group, though the substituent group is not necessarily required. The number of carbon atoms of the alkoxy group is not especially limited, but is usually in a range from 1 to 20. Further, if the number of carbon atoms of the alkoxy group is too large, the hole transportcapability may be impaired, and consequently more preferred is a methoxy group or ethoxy group.

The alkylthio group is an alkoxy group, the oxygen atom of the ether bond of which is substituted by a sulfur atom. The hydrocarbon group of the alkylthio group may have a substituent group, though the substituent group is not necessarily required. The number of carbon atoms of the alkylthio group is not especially limited, but is usually in a range from 1 to 20.

An aryl ether group denotes, for example, a functional group having an aromatic hydrocarbon group bonded via an ether bond such as a phenoxy group, and the aromatic hydrocarbon group may have a substituent group, though the substituent group is not necessarily required. The number of carbon atoms of the aryl ether group is not especially limited, but is usually in a range from 6 to 40.

The aryl thioether group is an aryl ether group, the oxygen atom of the ether bond of which is substituted by a sulfur atom. The aromatic hydrocarbon group in the aryl ether group may have a substituent group, though the substituent group is not necessarily required. The number of carbon atoms of the aryl ether group is not especially limited, but is usually in a range from 6 to 40.

The amino group may have a substituent group, though the substituent group is not necessary required. The substituent group may, for example, an alkyl group, aryl group, or heteroaryl group, etc. Any of these substituent groups may also further substituted.

The phosphine oxide group may also have a substituent group, though the substituent group is not necessarily required. The substituent group may, for example, an aryl group or heteroaryl group, etc. Any of these substituent groups may also be further substituted.

The silyl group denotes, for example, a functional group having a bond to a silicon atom such as a trimethylsilyl group, and the silyl group may have a substituent group, though the substituent group is not necessarily required. The number of carbon atoms of the silyl group is not especially limited, but is usually in a range from 3 to 20. Further, the number of silicon atoms is usually 1 to 6.

It is preferred that R¹ to R²⁰ each denote hydrogen and that R³ and R¹⁸ each denote a substituent group other than hydrogen from the viewpoints of easy availability of raw material, synthesis cost and hole transportcapability/electron-blockingcapability/high triplet level. In the case where R³ and R¹⁸ each denote a substituent group other than hydrogen, it is preferred that R³ and R¹⁸ denote, respectively independently, an alkyl group, cycloalkyl group, alkoxy group, aryl group or heteroaryl group. Above all, it is more preferred that R³ and R¹⁸ each denote an alkyl group, alkoxy group or aryl group. Further, it is also preferred that either of R³ and R¹⁸ denotes an N-phenylcarbazolyl group, to form carbazole tetrameter. In this case, the N-phenylcarbazolyl group may also be substituted by an alkyl group,cycloalkyl group, alkoxy group, aryl group or heteroaryl group. The explanation of these substituent groups is as described above.

Further, from the viewpoints of easy availability of raw material, synthesis cost and hole transportcapability/electron-blockingcapability/high triplet level, it is preferred that R ²¹ to R²³ each denote an aryl group or heteroaryl group. In the case where R²¹ to R²³ each denote an aryl group, a phenyl group or fluorenyl group is especially preferred.

The compounds represented by general formula (1) are not especially limited, but specifically the following compounds can be enumerated.

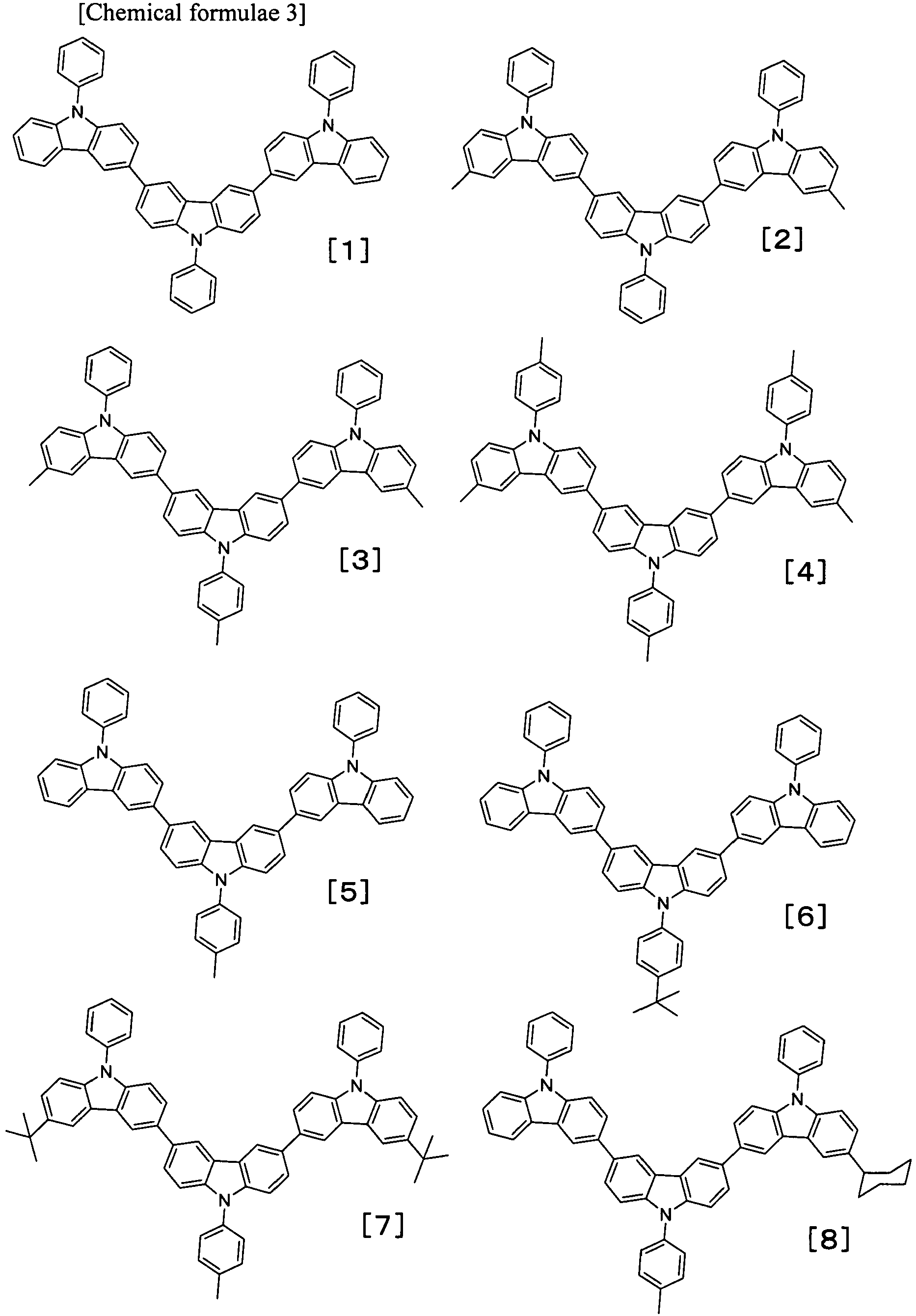

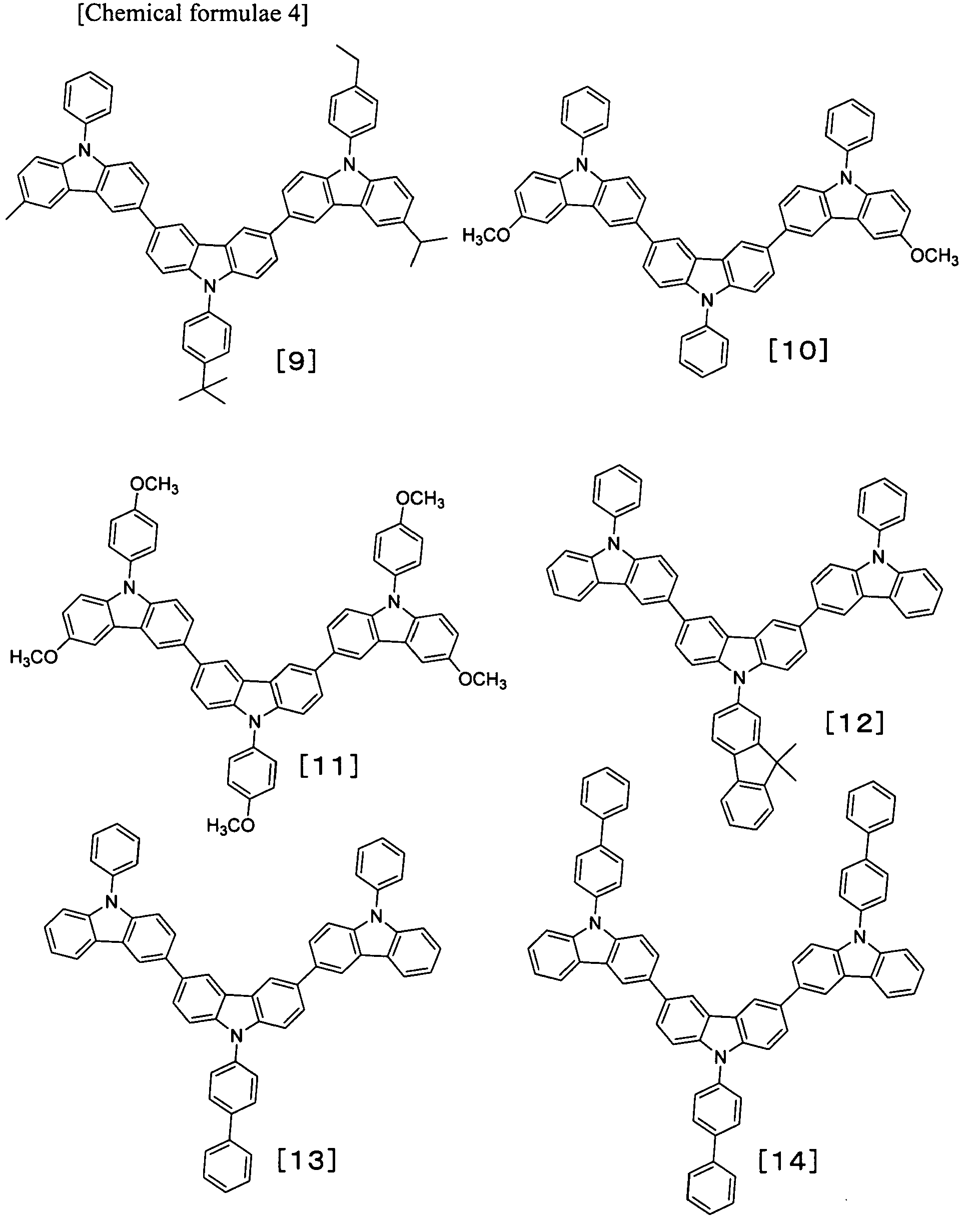

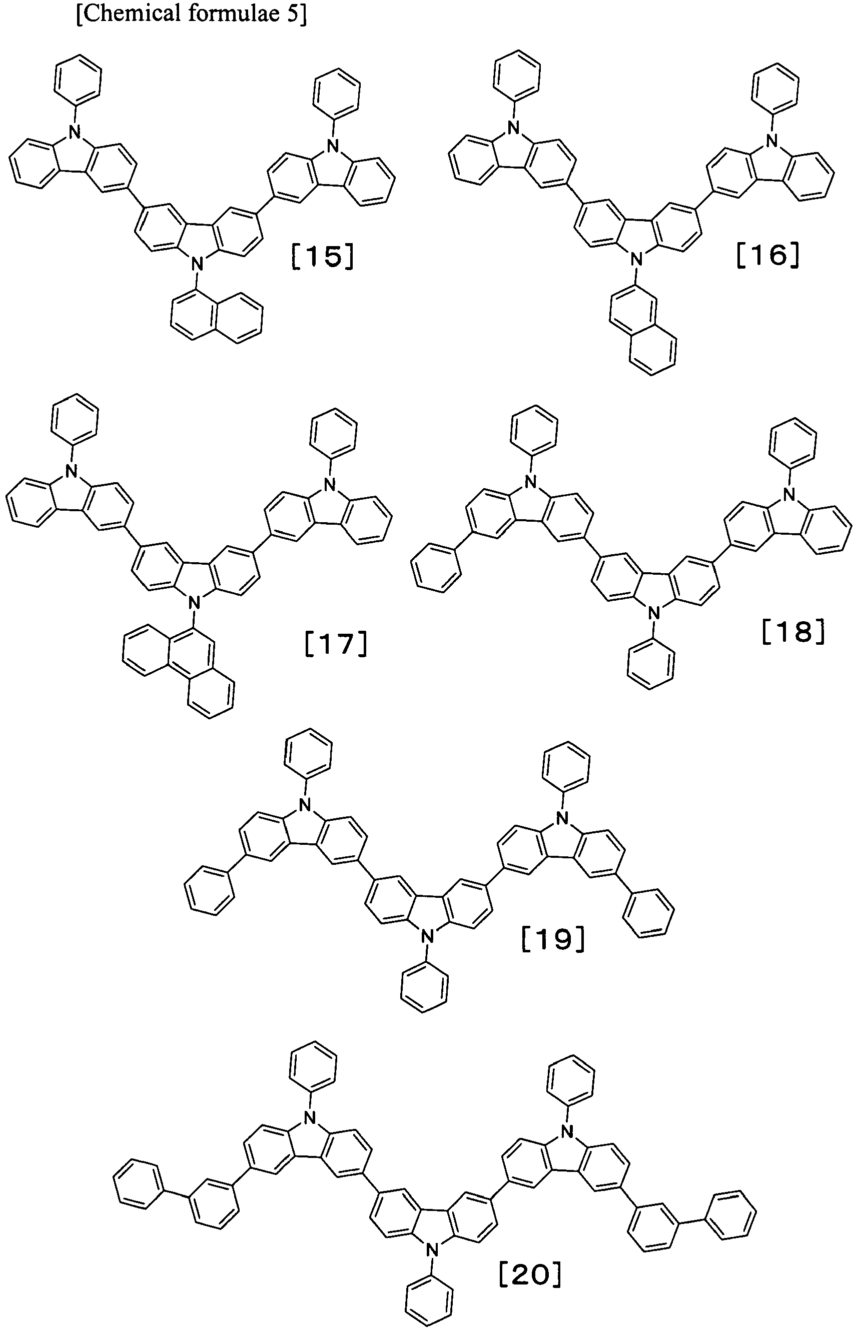

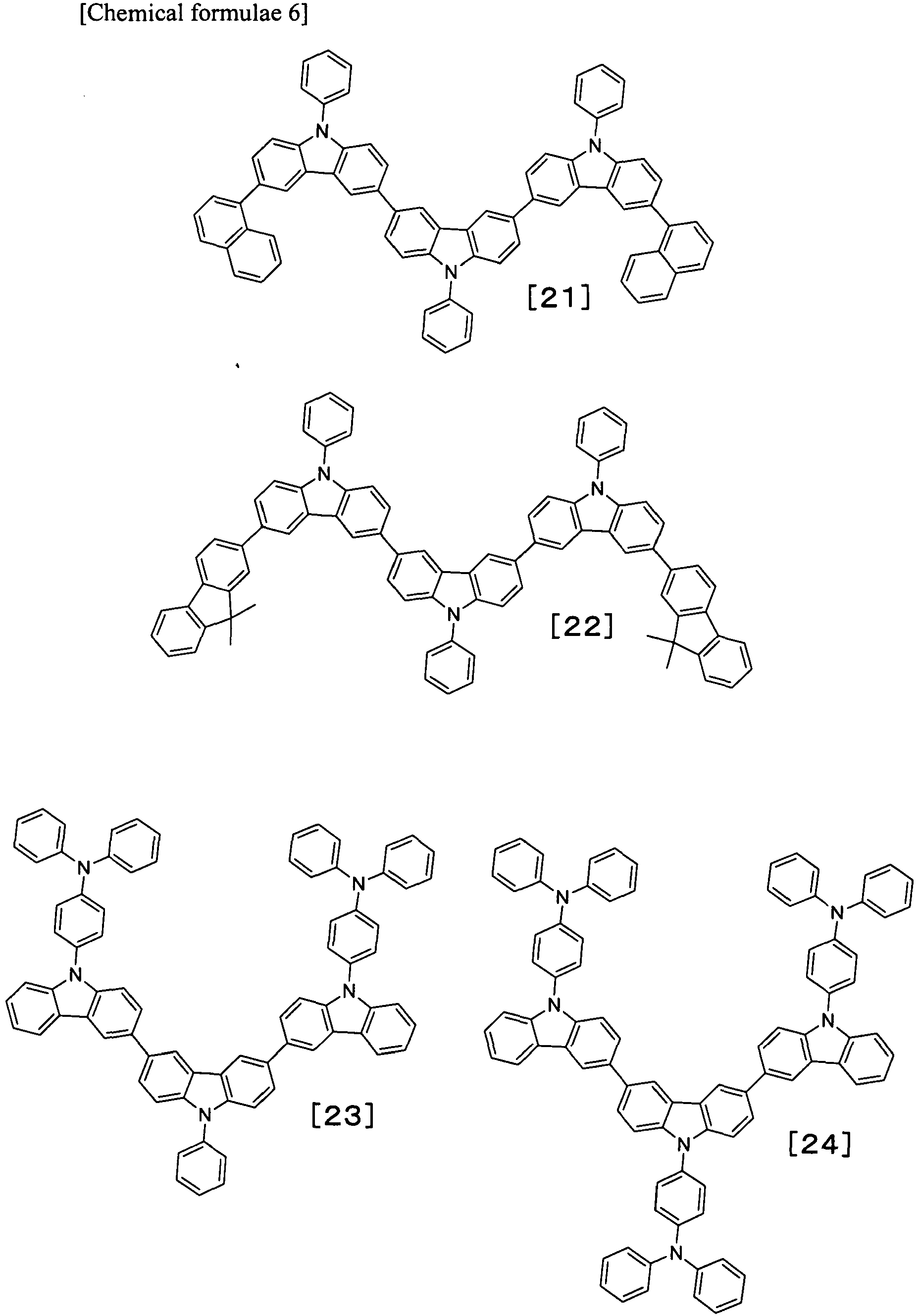

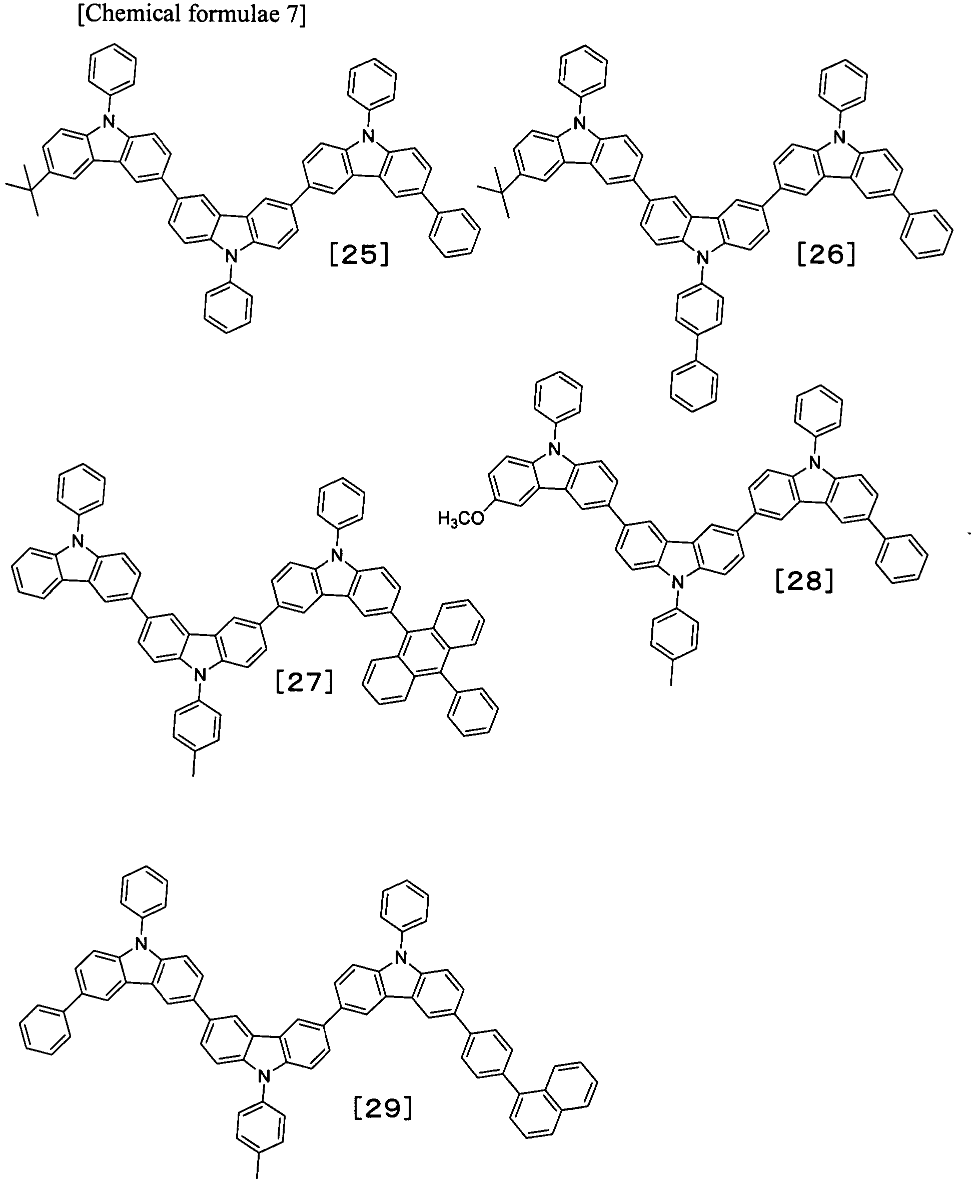

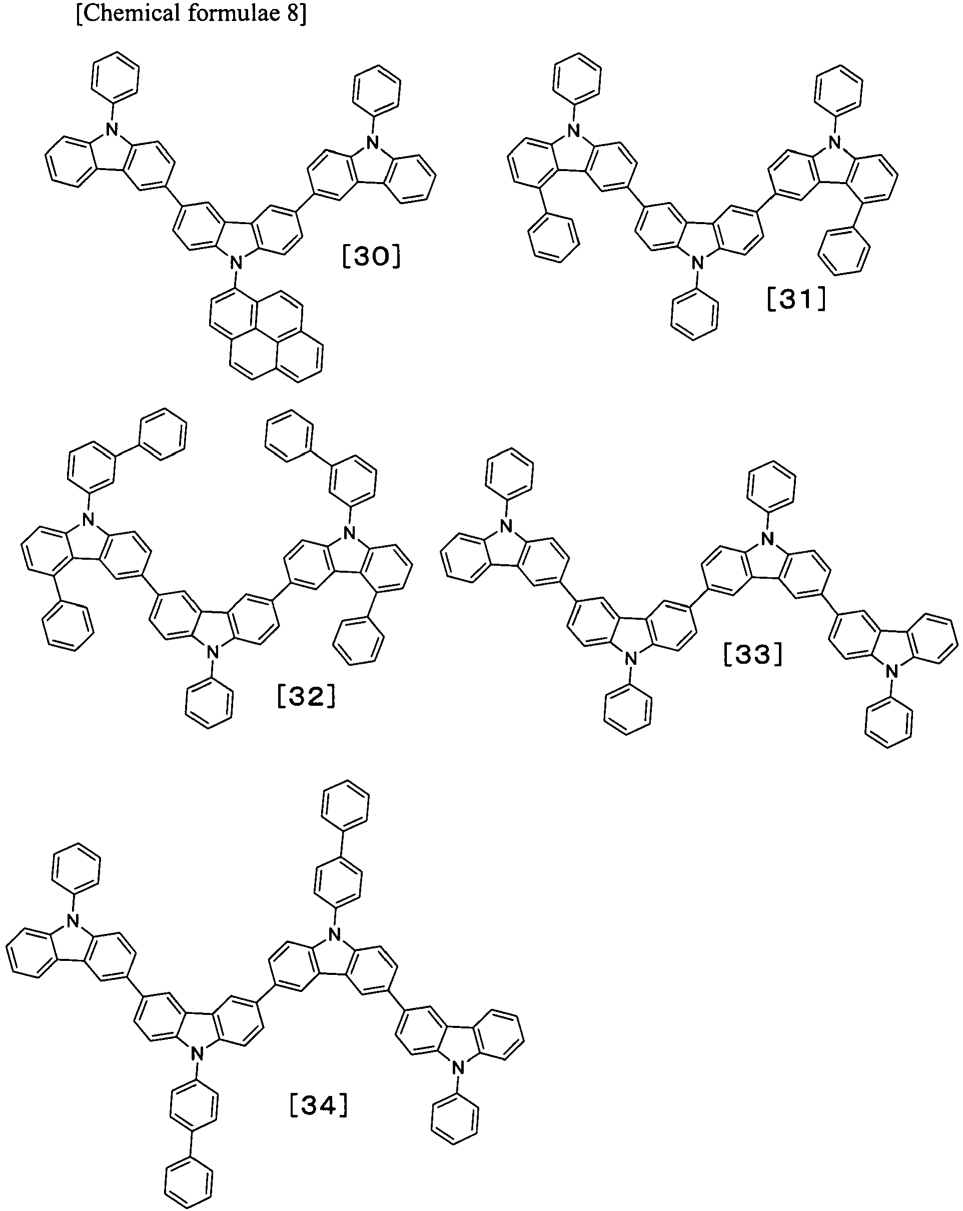

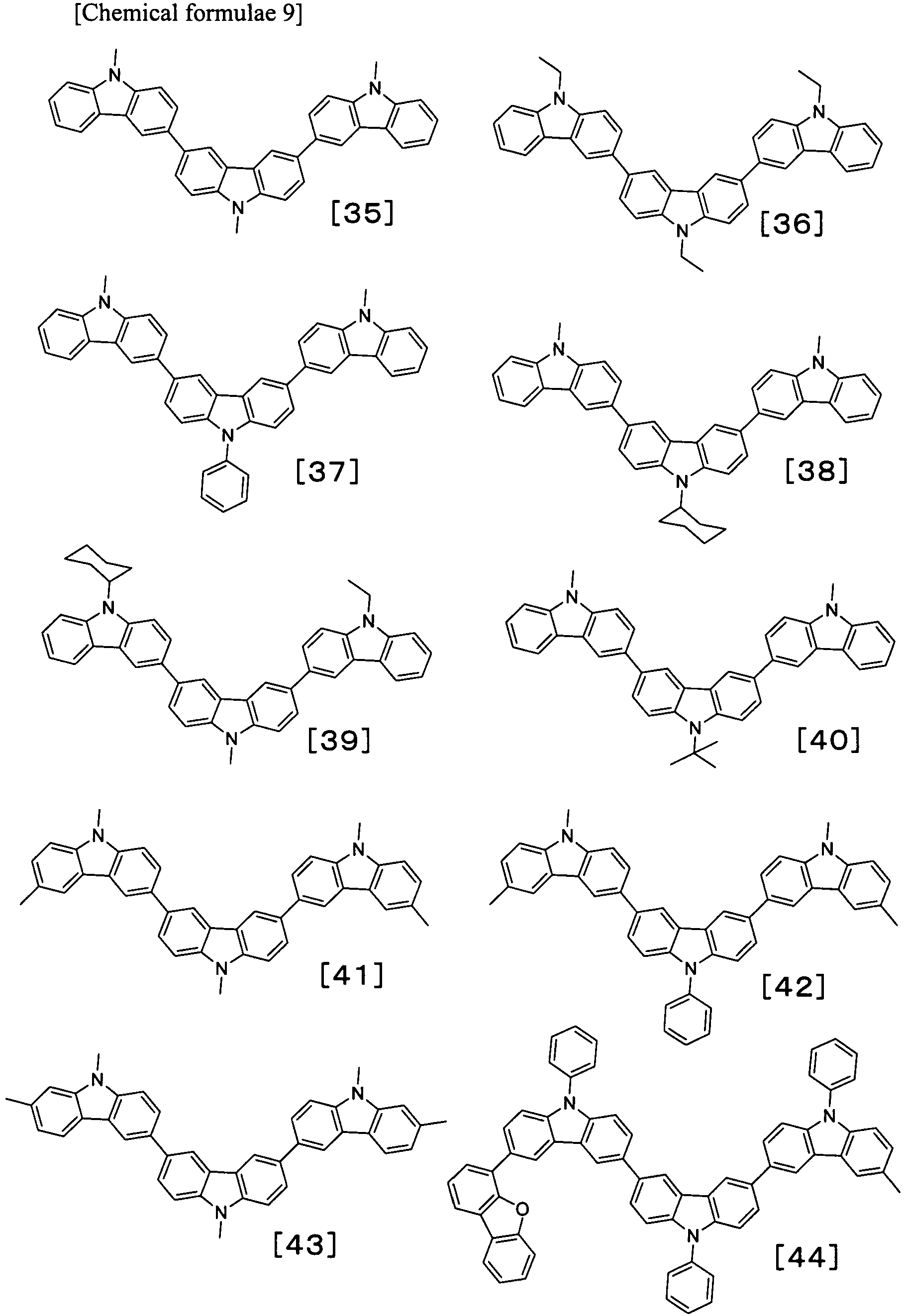

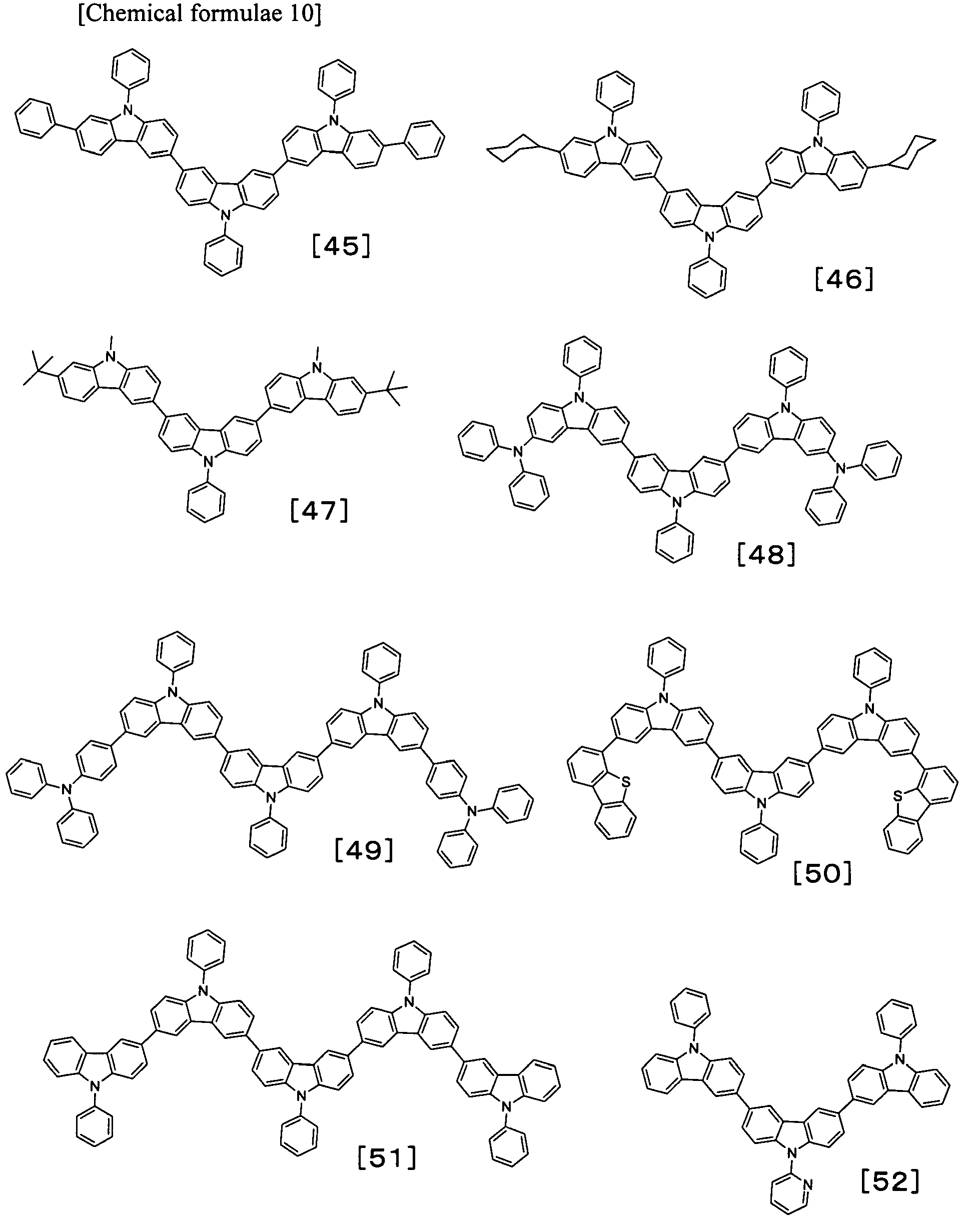

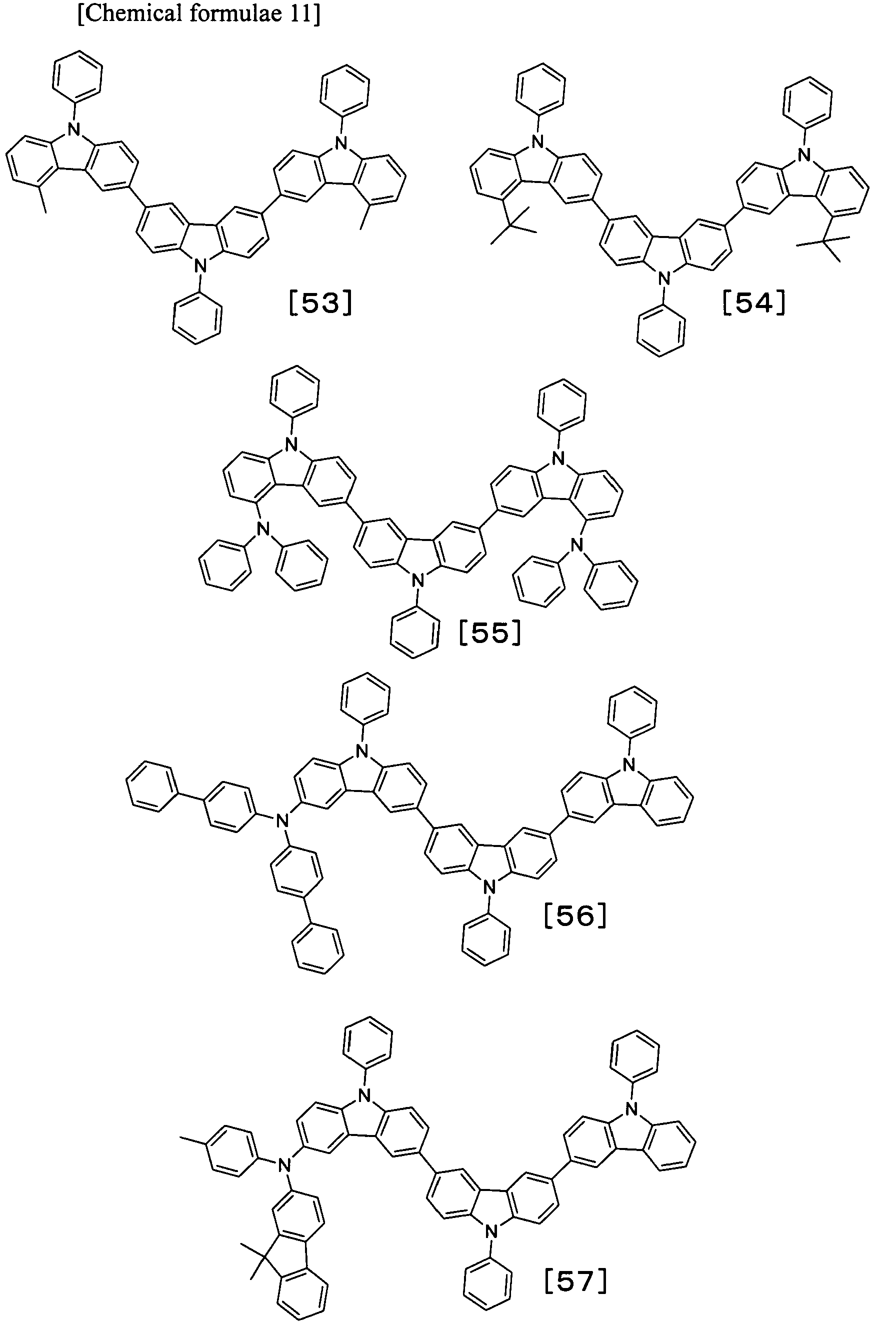

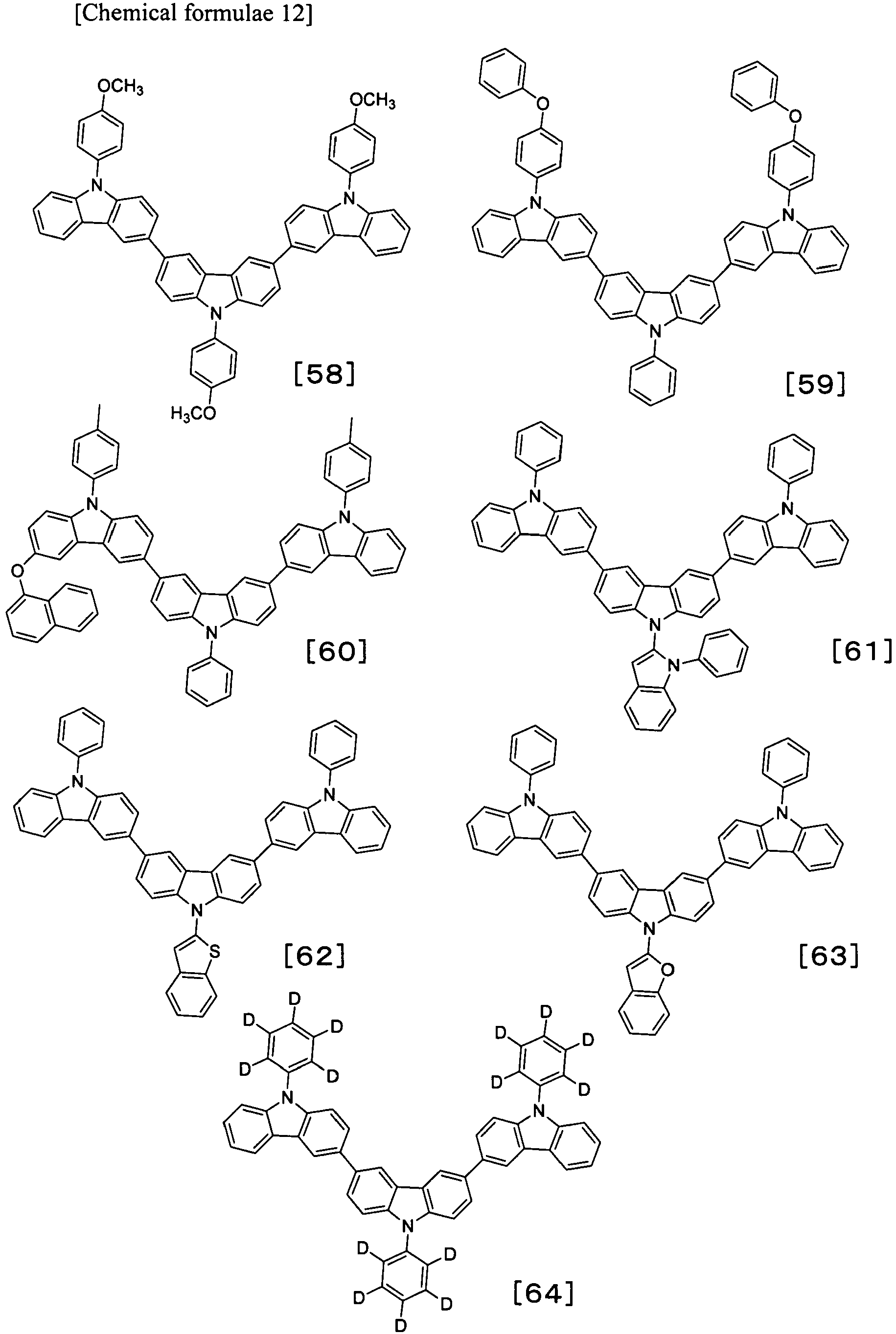

The compounds represented by general formula (1) can be produced by publicly known methods. That is, Suzuki coupling reaction between the dibromo compound of carbazole substituted at the 9-position and the monoboric acid of carbazole substituted at the 9-position can be used to easily synthesize the compounds, but the production method is not limited thereto.

Next, the modes for carrying out the light-emitting element of this invention are explained below in detail in reference to embodiments. The light-emitting element of this invention contains an anode, a cathode and at least a hole transport layer and an electron transport layer between said anode and said cathode.

The layer configuration between the anode and the cathode in such a light-emitting element can be a configuration comprising hole transport layer/light-emitting layer/electron transport layer, or a laminate configuration comprising hole injection layer/hole transport layer/light-emitting layer/electron transport layer, hole transport layer/light-emitting layer/electron transport layer/electron injection layer, or hole injection layer/hole transport layer/light-emitting layer/electron transport layer/electron injection layer. Further, each of these layers can also comprise a single layer or a plurality of layers.

The compound represented by general formula (1) is contained in the pore transport layer of the light-emitting element. The hole transport layer is a layer for transporting the holes injected from the anode to the light-emitting layer. The hole transport layer can comprise either one layer or a plurality of layers laminated. The compound represented by general formula (1) has high electron-blocking performance, and consequently in the case where the hole transport layer comprises a plurality of layers, it is preferred from the viewpoint of preventing the intrusion of electrons, that the hole transport layer containing the compound represented by general formula (1) is kept in direct contact with the light-emitting layer.

The pore transport layer may consist of the compound represented by general formula (1) only or may also have other materials mixed to such an extent that the effect of this invention is not impaired. In this case, the other materials used include, for example, benzidine derivatives such as 4,4'-bis(N-(3-methylphenyl)-N-phenylamino)biphenyl (TPD), 4,4'-bis(N-1-naphthyl)-N-phenylamino)biphenyl (NPD), 4,4'-bis(N,N-bis(4-biphenylyl)amino)biphenyl (TBDB), bis(N,N'-diphenyl-4-aminophenyl)-N,N-diphenyl-4,4'-diamino-1,1'-biphenyl (TPD232), materials called starburst arylamines such as 4,4',4"-tris(3-methylphenyl(phenyl)amino)triphenylamine (m-MTDATA) and 4,4',4"-tris(1-naphthyl(phenyl)amino)triphenylamine (1-TNATA), biscarbazole derivatives such as bis(N-arylcarbazole) and bis(N-alkylcarbazole), heterocyclic compounds such as pyrazoline derivatives, stilbene-based compounds, hydrazone-based compounds, benzofuranderivatives, thiophene derivatives, oxadiazole derivatives, phthalocyanine derivatives, and porphyrin derivatives, and polymers such as polycarbonates having any of the aforementioned monomers at the side chains, styrene derivatives, polythiophenes, polyanilines, polyfluorenes, polyvinylcarbazoles and polysilanes.

The electron transport layer of this invention is explained below. The electron transport layer is a layer for transporting the electrons injected from the cathode into the light-emitting layer.

The electron transport material used in the electron transport layer is not especially limited, and can be selected from compounds having a condensed polycyclic aromatic structure such as naphthalene, anthracene and pyrene, derivatives thereof, styryl-based aromatic ring derivatives typified by 4,4'-bis(diphenylethenyl)biphenyl, perylene derivatives, perynone derivatives, coumarin derivatives, naphthalimide derivatives, quinone derivatives such as anthraquinone and diphenoquinone, phosphorus oxide derivatives, carbazole derivatives and indole derivatives, quinolinol complexes such as tris(8-quinolinolate)aluminum (III), hydroxyazole complexes such as hydroxy-phenyl-oxazole complex, azomethine complexes, tropolone metal complexes and flavonol metal complexes. Since the drive voltage can be lowered, it is preferred to use a compound comprising elements selected from carbon, hydrogen, nitrogen, oxygen, silicon and phosphorus and having a heteroaryl ring structure containing an electron-acceptable nitrogen as the electron transport material.

The electron-acceptable nitrogen means a nitrogen atom forming a multiple bond with an adjacent atom. Since a nitrogen atom has high electron negativity, said multiple bond has electron-acceptable nature and is excellent in electron transport capability, and if the electron-acceptable nitrogen is used in the electron transport layer, the drive voltage of the light-emitting element can be lowered. Consequently the heteroaryl ring containing the electron-acceptable nitrogen has high electron affinity. As examples of the heteroaryl ring containing an electron-acceptable nitrogen, enumerated are a pyridine ring, pyrazine ring, pyrimidine ring, quinoline ring, quinoxaline ring, naphthyridine ring, pyrimidopyrimidine ring, benzoquinoline ring, phenanthroline ring, imidazole ring, oxazole ring, oxadiazole ring, triazole ring, thiazole ring, thiadiazole ring, benzoxazole ring, benzothiazole ring, benzimidazole ring, phenanthroimidazole ring, etc.

As preferred examples of the compounds having any of these heteroaryl ring structures, enumerated are benzimidazole derivatives, benzoxazole derivatives, benzthiazole derivatives, oxadiazole derivatives, thiadiazole derivatives, triazole derivatives, pyridine derivatives, pyrazine derivatives, phenanthroline derivatives, quinoxaline derivatives, quinoline derivatives, benzoquinoline derivatives, oligopyridine derivatives such as bipyridine and terpyridine, quinoxaline derivatives, naphthyridine derivatives, and phenanthroline derivatives. Among these derivatives, also considering electrochemical stability, benzimidazole derivatives, pyridine derivatives, quinoline derivatives, quinoxaline derivatives and phenanthroline derivatives are more preferred, and imidazole derivatives such as tris(N-phenylbenzimidazole-2-yl)benzene, oxadiazole derivatives such as 1,3-bis[(4-tert-butylphenyl)1,3,4-oxadiazolyl]phenylene, triazole derivatives such as N-naphthyl-2,5-diphenyl-1,3,4-triazole, phenanthroline derivatives such as Bathocuproin and 1,3-bis(1,10-phenanthroline-9-yl)benzene, benzoquinoline derivatives such as 2,2'-bis(benzo[h]quinoline-2-yl)-9,9'-spirobifluorene, bipyridine derivatives such as 2,5-bis(6'-(2',2"-bipyridyl))-1,1-dimethyl-3,4-diphenylsilole, terpyridine derivatives such as 1,3-bis(4'-(2,2':6'2"-terpyridinyl))benzene and naphthyridine derivatives such as bis(1-naphthyl)-4-(1,8-naphthyridine-2-yl)phenylphosphine oxide can be enumerated as preferred examples. Further, phenanthroline derivatives such as 1,3-bis(1,10-phenanthroline-9-yl)benzene in which a plurality of phenanthroline skeletons are connected by using any of double bonds, triple bonds, substituted or non-substituted aromatic hydrocarbon residues, substituted or non-substituted aromatic heterocyclic residues and combinations thereof as connection units are especially preferred, since these derivatives have high electron injection/transport properties.

Further, a preferred electron transport material can also be a compound containing a condensed polycyclic aromatic hydrocarbon as a basic skeleton and containing the abovementioned electron-acceptable nitrogen-containing heteroaryl ring as a substituent group. It is preferred to have a condensed polycyclic aromatic hydrocarbon skeleton for such reasons that the glass transition temperature can be enhanced and that the electron mobility becomes also so large as to enhance the effect of lowering the voltage of the light-emitting element. Further, considering the longer durability life of the element owing to stable thin-film formability, easy synthesis and easy raw material availability, it is especially preferred that the condensed polycyclic aromatic hydrocarbon skeleton is an anthracene skeleton or pyrene skeleton. As preferred examples, enumerated are a pyrene derivative substituted at the 1- and 3-positions by an alkyl group, aryl group or heteroaryl group, a pyrene derivative substituted at the 1-, 3- and 7-positions by an alkyl group, aryl group orheteroaryl group, a pyrene derivative substituted at the 1- and 6-positions by an alkyl group, aryl group or heteroaryl group, an anthracene derivative substituted at the 1- and 10-positions by an alkyl group, aryl group or heteroaryl group, etc. Any one of the abovementioned electron transport materials can be used alone, but two or more of the electron transport materials can be used as a mixture, or one or more other electron transport materials can also be mixed with any of the abovementioned electron transport materials.

Furthermore, the electron transport layer of this invention contains a donor compound. In the case where such an electron transport layer and a hole transport layer having any of the compounds represented by general formula (1) are combined, a light-emitting element driven at a low voltage and having high efficiency and long life can be obtained.

In general, in order to obtain a light-emitting element having highly efficient light emission and long life, it is necessary to recombine electrons and holes without allowing them to leak from the light-emitting layer. Therefore, the pore transport material is required to have the performance of allowing holes to be smoothly injected from the anode and transporting the holes smoothly to the light-emitting layer (hole injection and transport capabilities) and also to have the performance of confining the electrons flowing in counter-current within the light-emitting layer (electron-blockingcapability). In the case where the electron-blockingcapability is poor, the electrons intrude even into the hole transport layer, to lower light emission efficiency and to deteriorate the hole transport material, thus shortening the durability life of the element.

The difference in electronic blocking capability is mainly caused by the energy of LUMO (Lowest Unoccupied Molecular Orbital) level of the hole transport material. That is, if the energy level of LUMO level is shallower, the electron affinity of the hole transport material becomes smaller, and the intrusion of electrons into the hole transport material can be more easily prevented. The hole transport material having a LUMO level of a shallow energy level like this is considered to have excellent electron-blockingcapability.

More detailed explanation is made. Fig. 1 simply illustrates the energy states of the charges inside an organic electroluminescent element. For simplification of explanation, two-layer element comprising a hole transport layer and a light-emitting layer without an electron transport layer is shown. Symbol 1 denotes the energy level of the anode (work function), and 2 denotes the energy level of the HOMO level of the hole transport material, 3 denoting the energy level of the HOMO level of the light-emitting layer. Symbol 4 denotes the energy level of the cathode (work function), and 5 denotes the energy level of the LUMO level of the light-emitting layer, and 6 denoting the energy level of the LUMO level of the hole transport material. Symbol 7 denotes the line indicating the interface between the anode and the hole transport layer, and 8 denotes the line indicating the interface between the hole transport layer and the light-emitting layer, and 9 denoting the line indicating the interface between the light-emitting layer and the cathode. Symbol 11 denotes a hole on the anode, and 12 denotes a hole injected into the hole transport material, and 13 denoting a hole injected into the light-emitting layer. Symbol 14 denotes an electron on the cathode, and 15 denotes an electron injected into the light-emitting layer. Symbol 16 denotes the line indicating the recombination between an electron and a hole, and 17 denotes the energy difference between the LUMO level of the light-emitting layer and the LUMO level of the hole transport layer. With regard to a shallow energy level and a deep energy level, for example, in the case of LUMO level, the symbol 5 of Fig. 1, if written upward in the drawing, means a shallow level, and the symbol 5 of Fig. 1, if written downward, means a deep level.

If a voltage is applied between the anode and the cathode, the hole (11) on the anode is injected into the HOMO level (2) of the hole transport layer. Likewise, the electron (14) on the cathode is injected into the LUMO level (5) of the light-emitting layer. Further according to the applied voltage, the hole (12) and the electron (15) move toward counter electrodes. Furthermore, the hole (12) is injected into the light-emitting layer, and within the light-emitting layer, the hole (13) and the electron (15) recombine to produce an excited state of the light-emitting material. Using the light discharge in the excited state is the basic action mechanism of the organic electroluminescent element. Ideally holes and electrons recombine at 1:1 in the light-emitting layer, but actually, either of the carriers is excessive in most cases. Let's consider a case where electrons are excessive. The electrons not participating in the recombination are considered to go further from the light-emitting layer and to climb over the energy difference (17) (i.e., electron injection barrier) between the LUMO level of the light-emitting layer and the LUMO level of the hole transport layer, intruding into the hole transport layer. In this case, if the LUMO level (6) of the hole transport material is deeper, in other words, if the electron injection barrier expressed by (17) is smaller, the electrons are likely to intrude into the hole transport layer. On the contrary, if the LUMO level (6) is shallower, in other words, if the electron injection barrier (17) is larger, electrons are unlikely to intrude into the hole transport layer, and it be said that the electron-blocking capability is high. Therefore, the energy difference (17) between the LUMO level of the light-emitting layer and the LUMO level of the hole transport layer greatly affects the abovementioned electron-blocking capability of the hole transport material.

Many of the conventional hole transport materials have good hole injection and transport capabilities, but most of them are insufficient in electron-blocking capability. The reason is that the LUMO level (6) of the hole transport material in Fig. 1 is deep, and that the electron injection barrier (17) is small. For example, the LUMO level of a benzidine-based hole transport material often used hitherto is 2.3 to 2.4 eV, considering that the energy level of HOMO level is approximately 5.3 to 5.4 eV and that the energy gap between HOMO and LUMO is approximately 3.0 eV On the contrary, the HOMO level of a compound represented by general formula (1) is approximately 5.3 to 5.4 eV, and the energy gap is as wide as approximately 3.3 eV. Therefore, it can be considered that the compound has a LUMO level of 2.0 to 2.1 eV shallower than that of the conventional material and has high electron blocking capability. Meanwhile, the LUMO level in this case refers to the numerical value obtained by adding the value of the energy gap estimated from the wavelength at the UV absorption end of the vapor-deposited film to the HOMO level that is the ionization potential measured by a photoelectron spectrometer, AC-2 (produced by Riken Keiki Co., Ltd.) in atmosphere. All the HOMO levels and energy gaps and LUMO levels stated hereunder are the values obtained by the same method.

Considering the abovementioned matters, in the case where an electron transport layer containing a donor compound is used to enhance the electron injection and transport capabilities in an attempt to drive the element at a low voltage, the electrons in the light-emitting layer become excessive and are going to intrude into the hole transport layer. However, since the compound represented by general formula (1) has high electron-blocking capability as described above, the intrusion of electrons into the hole transport layer can be prevented. Thus, it can be considered that while the effect of driving at a low voltage owing to the electron transport layer containing a donor compound is obtained, a light-emitting element with high light emission efficiency and long life can be obtained.

On the other hand, as conventional hole transport materials, biscarbazole derivatives are also known, but the HOMO levels of these compounds are approximately 5.5 to 5.6 eV, and the energy gaps are approximately 3.3 eV As a result of them, the LUMO levels of biscarbazole derivatives are approximately 2.2 to 2.3 eV and are also deeper than those of the compounds represented by general formula (1). For this reason, biscarbazole derivatives are also considered to be poor in electron-blocking capability. Further, the HOMO levels of biscarbazole derivatives are also deeper than those of the compounds represented by general formula (1), and it can be considered that the compounds represented by general formula (1) are excellent also from the viewpoint of easy injection of holes from the anode.

A donor compound in this invention is a compound that facilitates the injection of electrons from the cathode or electron injection layer into the electron transport layer by improving the electron injection barrier, and that further enhances the electric conductivity of the electron transport layer.

As preferred examples of the donor compound in this invention, enumerated are alkali metals, inorganic salts containing alkali metals, complexes between alkali metals and organic substances, alkaline earth metals, inorganic salts containing alkaline earth metals, complexes between alkaline earth metals and organic substances, etc. Metals preferred as the alkali metals and the alkaline earth metals include alkali metals such as lithium, sodium, potassium, rubidium and cesium and alkaline earth metals such as magnesium, calcium, cerium and barium, each of which has a large effect of enhancing electron transport capability at low work function.

Further, in view of easy vapor deposition in vacuum and excellent handling, inorganic salts and complexes with organic substances are preferred to metals alone. Furthermore, in view of easy handling in atmosphere and easy control of added concentration, complexes with organic substances are more preferred. Examples of the inorganic salts include oxides such as LiO and Li₂O, nitrides, fluorides such as LiF, NaF and KF, carbonates such as Li₂CO₃, Na₂CO₃, K₂CO₃, Rb₂CO₃ and C_{S2}CO₃, etc. Moreover, as preferred examples of the alkali metals and the alkaline earth metals, from the viewpoint of obtaining a large low-voltage drive effect, enumerated are lithium and cesium. Further, as preferred examples of the organic substances in the complexes with organic substances, enumerated are quinolinol, benzoquinolinol, pyridylphenol, flavonol, hydroxyimidazopyridine, hydroxybenzoazole, hydroxytriazole, etc. Among them, from the viewpoint of obtaining a large effect of lowering the voltage of the light-emitting element, complexes between alkali metals and organic substances are preferred, and further from the viewpoints of easy synthesis and thermal stability, complexes between lithium and organic substances are preferred. Lithium quinolinol available at a relatively low price is especially preferred.

Further, the electron transport layer may contain two or more donor compounds. A suitable doping concentration depends on the material and the film thickness of the doping region, and it is preferred that the wt% ratio of an organic compound and a donor compound is in a range from 100:1 1 to 1:100. A more preferred range is 10:1 1 to 1:10.

Furthermore, an electron transport layer containing such a donor compound can also be used as a single electron transport layer. Otherwise, the electron transport layer can also be provided as two layers, and if the layer kept in contact with the light-emitting layer is called the first electron transport layer, while the layer not kept in contact is called the second electron transport layer, the first electron transport layer can be made not to contain a donor compound while the second electron transport layer can be made to contain a donor compound. However, in the case where the donor compound is an inorganic material such as an alkali metal, alkaline earth metal or any of oxides thereof, nitrides thereof, fluorides thereof and carbonates thereof, if a layer containing any of them is kept in direct contact with the light-emitting layer, the light-emitting layer may be quenched to lower light emission efficiency. Therefore, a configuration wherein the layer not containing the donor compound is used as the first electron transport layer is preferred. Further, in this case, the electron transport materials of the two layers can be identical to or different from each other.

Moreover, the electron transport layer containing a donor compound of this invention can also be suitably used forthe charge-generation layers in a tandem structure element obtained by connecting a plurality of light-emitting elements.

As the electron transport material combined with a donor compound, a material containing an electron-acceptable nitrogen as described above can also be used. Even a material not containing an electron-acceptable nitrogen can be used if the conductivity and the electron injection and transport capabilities of the material can be enhanced by adding a donor compound to the material. Preferred examples include compounds having an anthracene skeleton or pyrene skeleton.

Theelectron transport material that can be combined with a donor compound is not especially limited, but specifically the following examples can be enumerated.

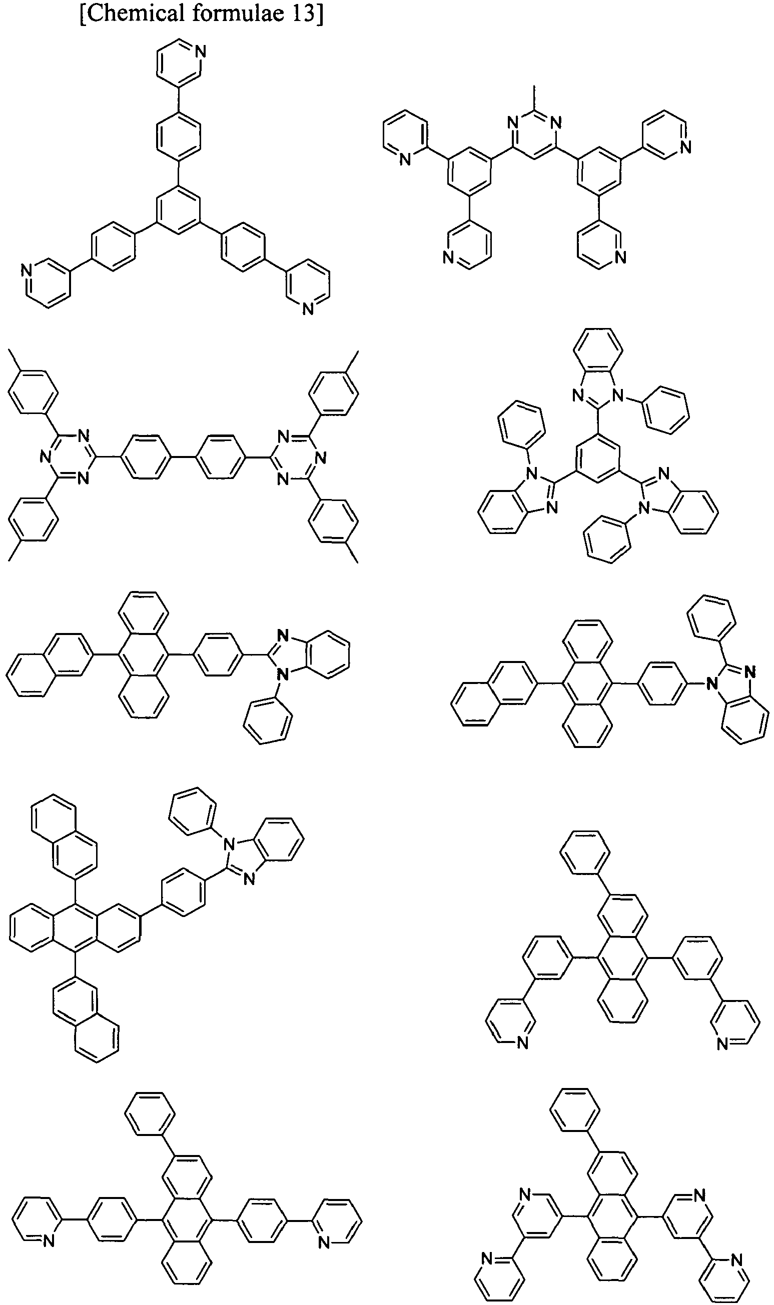

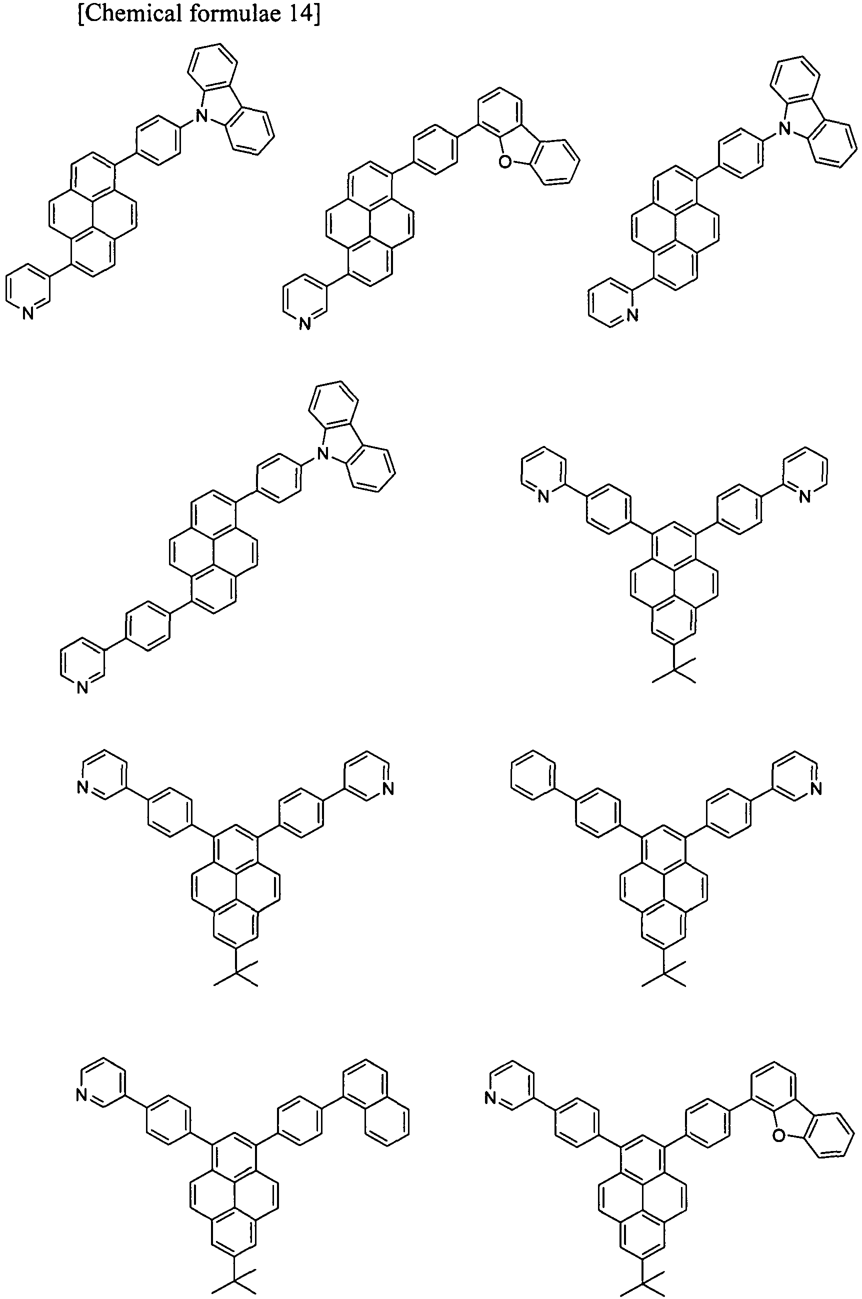

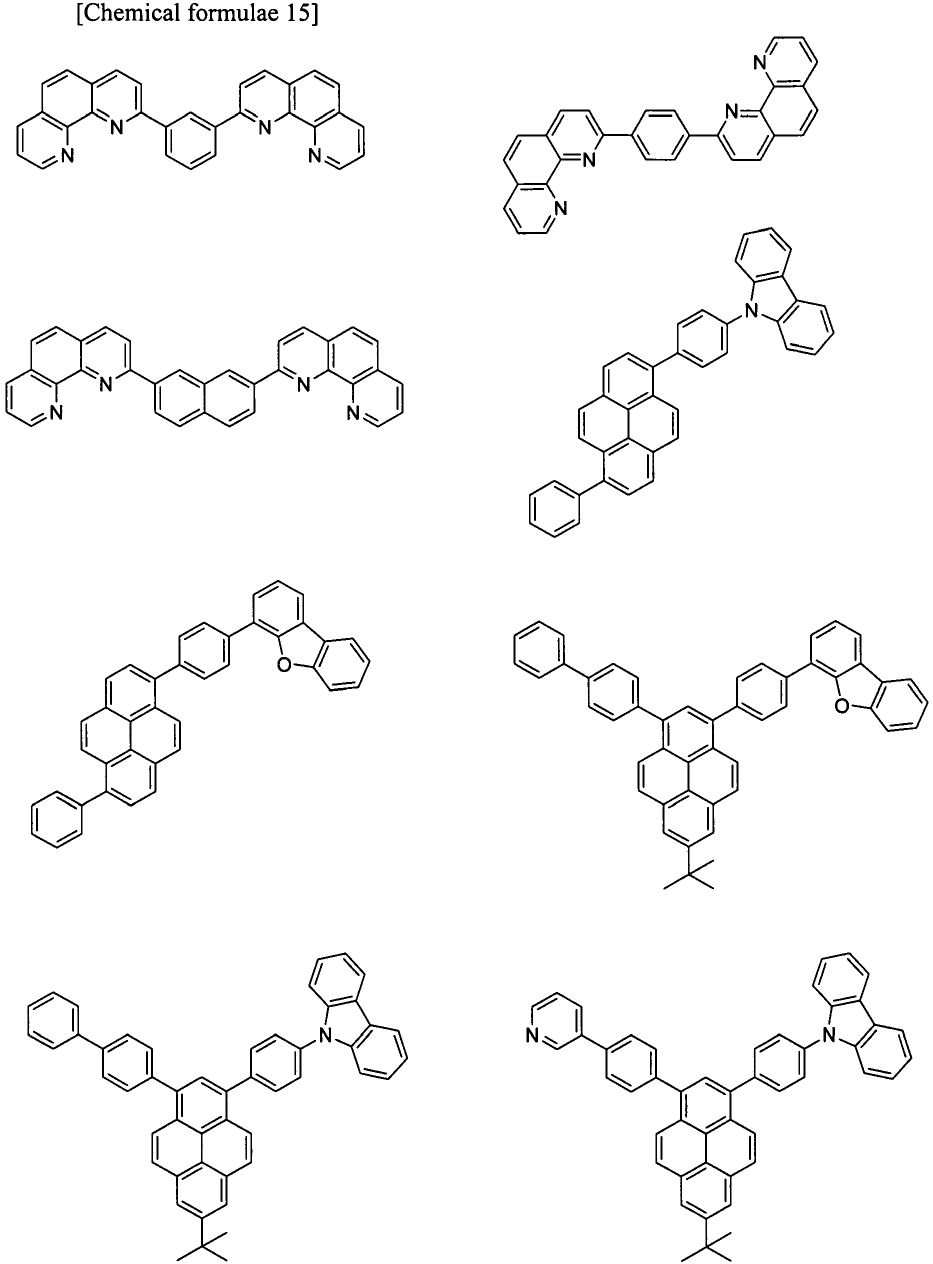

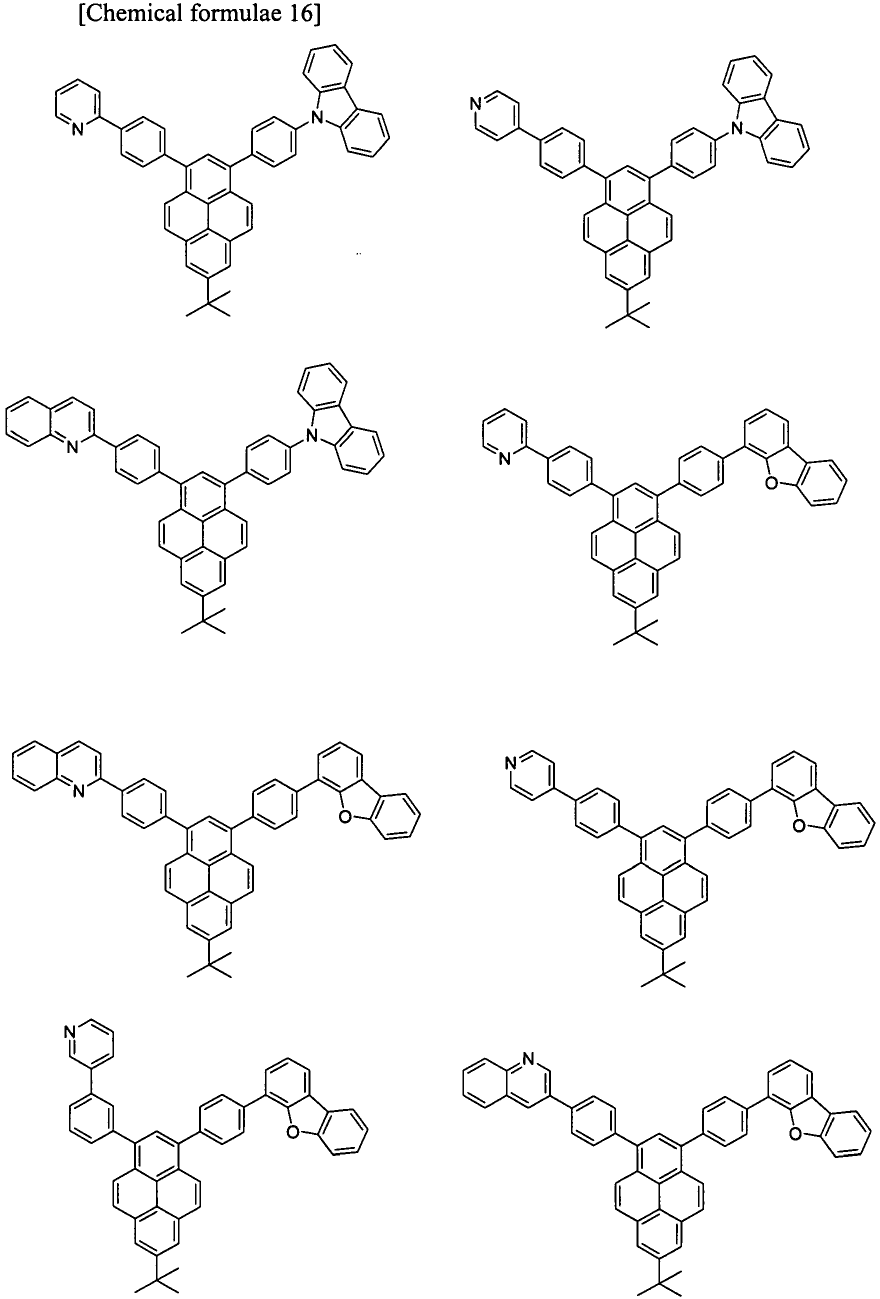

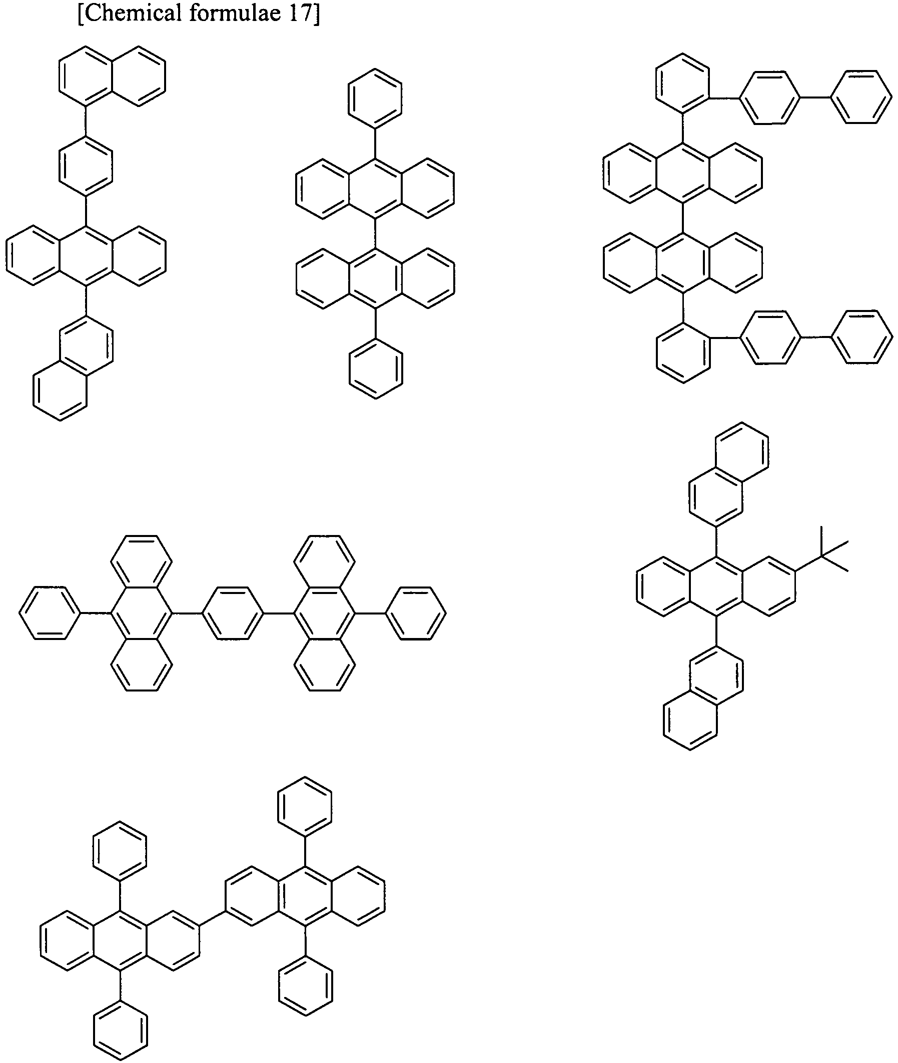

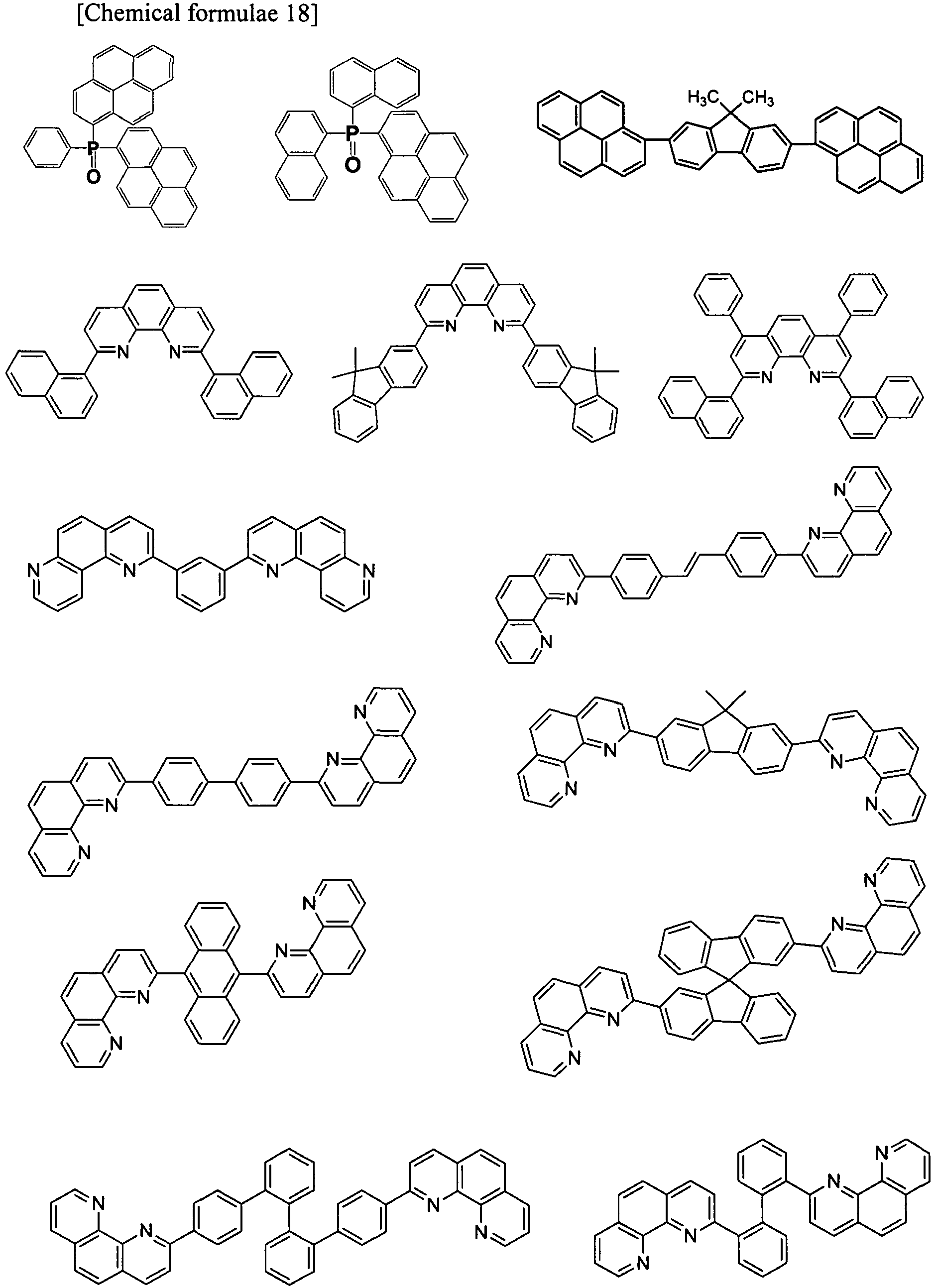

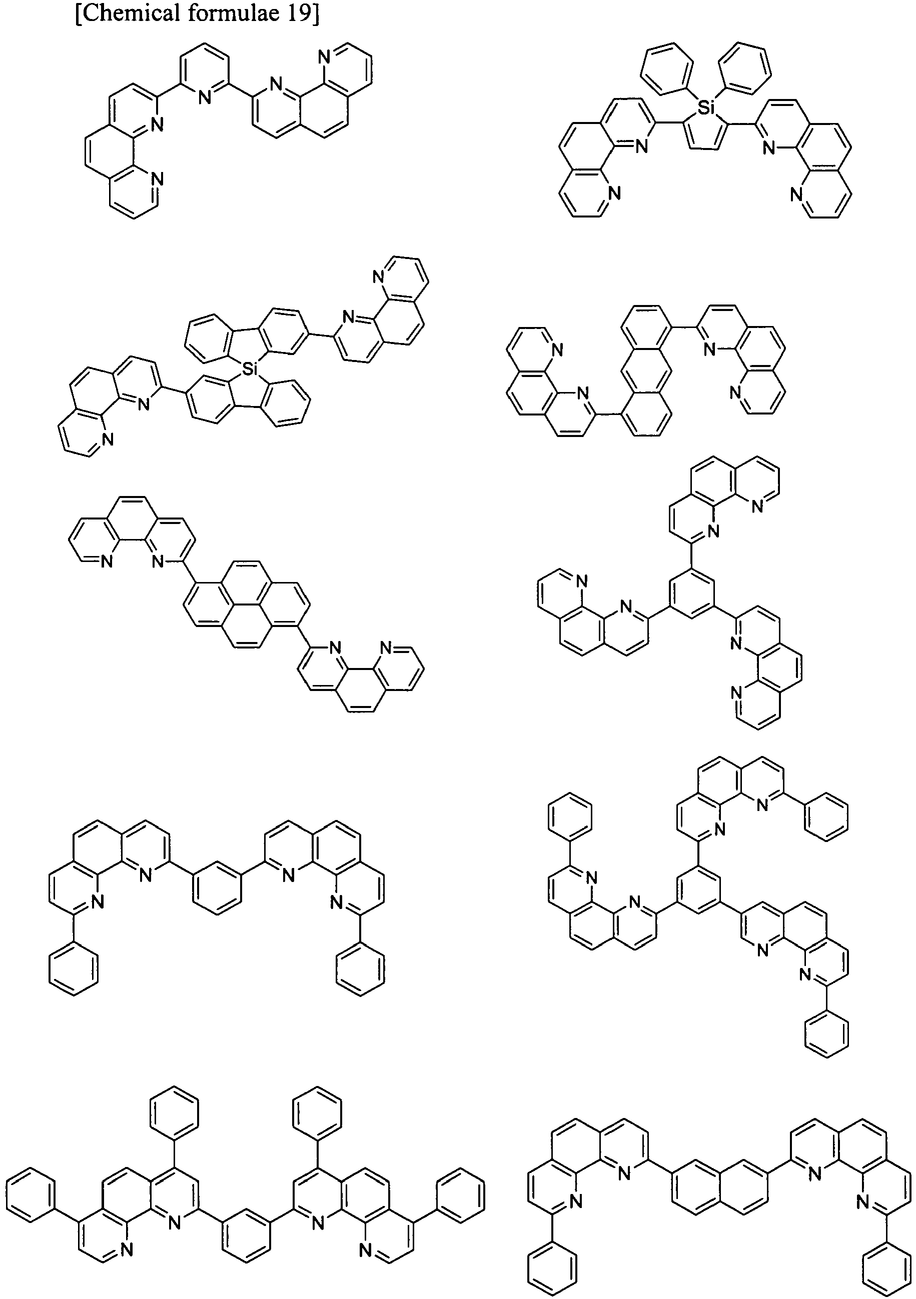

The hole injection layer is inserted between the anode and the hole transport layer. The hole injection layer can be either a single layer or a plurality of layers laminated. It is preferred that the hole injection layer exists between the hole transport layer containing the compound represented by general formula (1) and the anode since the light-emitting element can be driven at a lower voltage, can have a longer durability life, and can be enhanced in the carrier balance of the element, to also enhance the light emission efficiency.

The material used as the hole injection layer is not especially limited, and as examples of the material, enumerated are benzidine derivatives such as 4,4'-bis(N-3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB) and bis(N,N'-diphenyl-4-aminophenyl)-N,N-diphenyl-4,4'-diamino- 1, 1'-biphenyl (TPD232), materials called starburst arylamines such as 4,4',4"-tris(3-methylphenyl(phenyl)amino)triphenylamine (m-MTDATA) and 4,4',4"-tris(1-naphthyl(phenyl)amino)triphenylamine (1-TNATA), biscarbazole derivatives such as bis(N-arylcarbazole) and bis(N-alkylcarbazole), heterocyclic compounds such as pyrazoline derivatives, stilbene-based compounds, hydrazone-based compounds, benzofuran derivatives, thiophene derivatives, oxadiazole derivatives, phthalocyanine derivatives, and porphyrin derivatives, polymers such as polycarbonates having any of the aforementioned monomers at the side chains, styrene derivatives, polythiophenes, polyanilines, polyfluorenes, polyvinylcarbazoles and polysilanes. Further, the compounds represented by general formula (1) can also be used. Above all, from the viewpoints of having a HOMO level shallower than those of the compounds represented by general formula (1) and smoothly injecting and transporting holes from the anode to the hole transport layer, benzidine derivatives and starburst arylamine-based materials can be more preferably used.

Any one of these materials can be used alone, or two or more of the materials can also be used as a mixture. Otherwise, a plurality of materials can also be laminated to form the hole injection layer. Further, it is preferred that the hole injection layer consists of an acceptor compound alone or comprises any of the abovementioned hole injection materials doped with an acceptor compound, since the abovementioned effects can be remarkably obtained. An acceptor compound is a material forming a charge-transfer complex together with the hole transport layer the acceptor compound is kept in contact with in the case where the acceptor compound is used as a single-layer film. In the case where the acceptor compound is used as a dopant, the acceptor compound is a material forming a charge-transfer complex together with the material constituting the hole injection layer. If such a material is used, such effects as enhancing the conductivity of the hole injection layer for contribution to lowering the drive voltage of the element, enhancing the light emission efficiency, and elongating the durability life can be obtained.

As examples of the acceptor compound, enumerated are metal chlorides such as iron (III) chloride, aluminum chloride, gallium chloride, indium chloride and antimony chloride, metal oxides such as molybdenum oxide, vanadium oxide, tungsten oxide and ruthenium oxide, and charge-transfer complexes such as tris(4-boromophenyl)aluminum hexachloroantimonate (TBPAH). Further, organic compounds having a nitro group, cyano group, halogen or trifluoromethyl group in the molecule, quinone-based compounds, acid anhydride-based compounds, fullerenes and the like can also be suitably used. Specific examples of these compounds include hexacyanobutadiene, hexacyanobenzene, tetracyanoethylene, tetracyanoquinodimethane (TCNQ), tetrafluorotetracyanoquinodimethane (F4-TCNQ), 2,3,6,7,10,11-hexacyano-1,4,5,8,9,12-hexaazatriphenylene (HAT CN6), p-fluoranil, p-chloranil, p-bromanil, p-benzoquinone, 2,6-dichlorobenzoquinone, 2,5-dichlorobenzoquinone, tetramethylbenzoquinone, 1,2,4,5-tetracyanobenzene, o-dicyanobenzene, p-dicyanobenzene, 1,4-dicyanotetrafluorobenzene, 2,3-dichloro-5,6-dicyanobenzoquinone, p-dinitrobenzene, m-dinitrobenzene, o-dinitrobenzene, p-cyanonitrobenzene, m-cyanonitrobenzene, o-cyanonitrobenzene, 1,4-naphthoquinone, 2,3-dichloronaphthoquinone, 1-nitronaphthalene, 2-nitronaphthalene, 1,3-dinitronaphthalene, 1,5-dinitronaphthalene, 9-cyanoanthracene, 9-nitroanthracene, 9,10-anthraquinone, 1,3,6,8-tetranitrocarbazole, 2,4,7-trinitro-9-fluorenone, 2,3,5,6-tetracyanopyridine, maleic anhydride, phthalic anhydride, C60 and C70, the following compounds, etc.

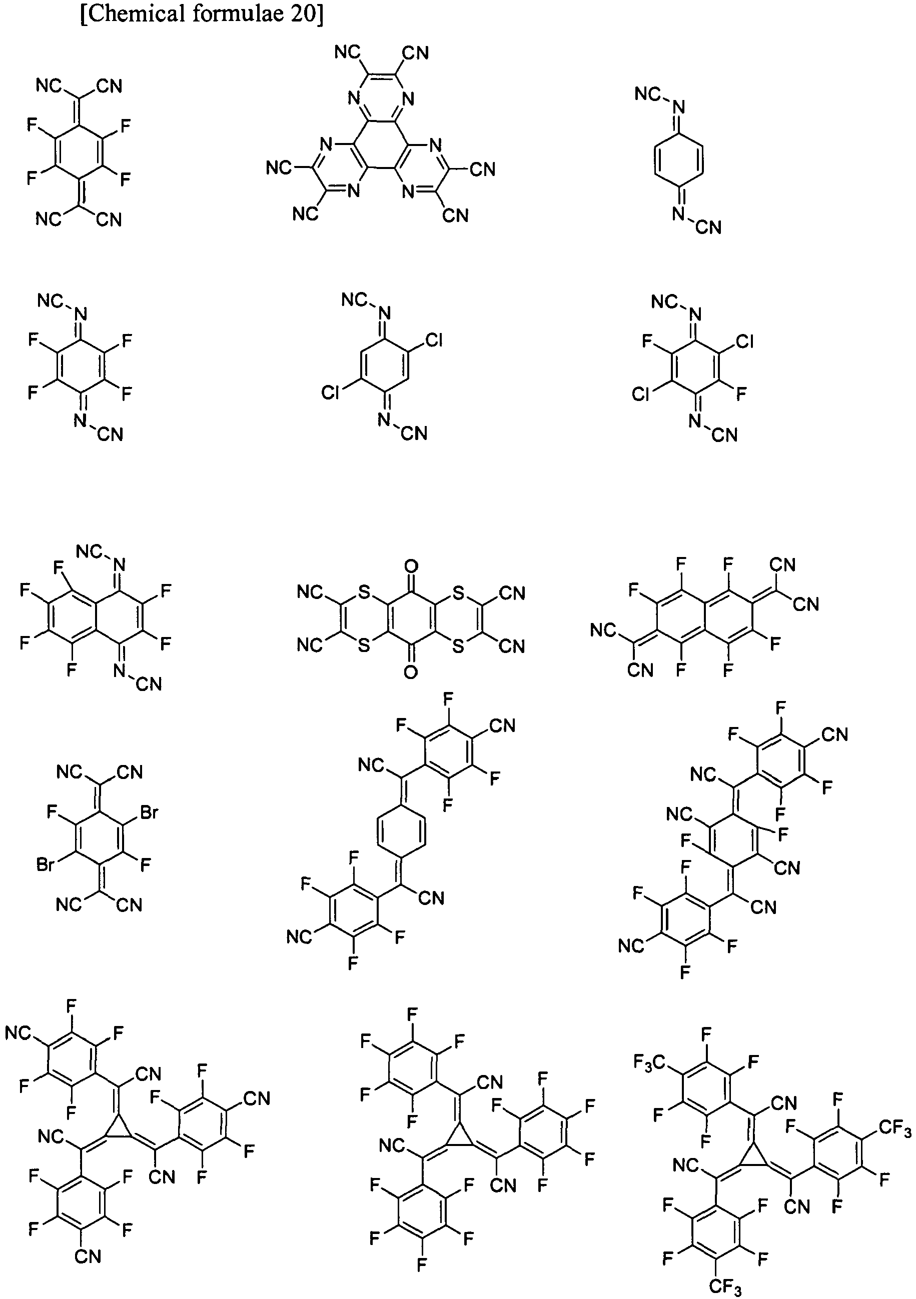

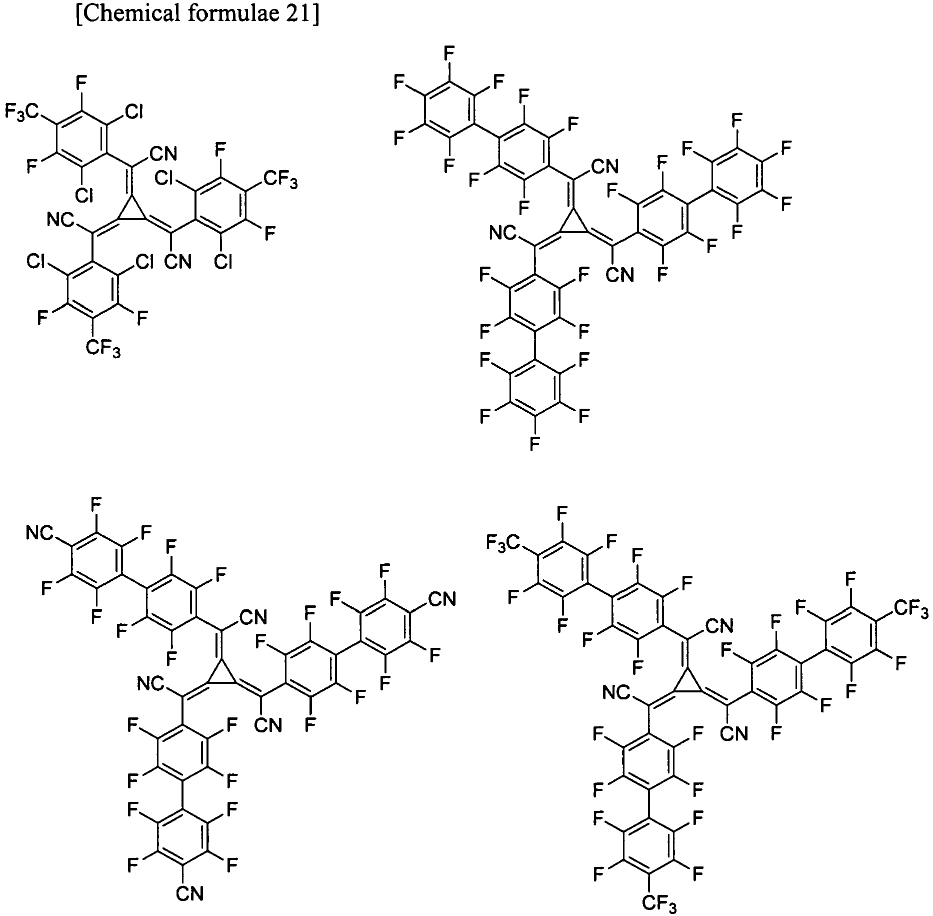

Among them, metal oxides and cyano group-containing compounds are preferred for such reasons that handling is easy, that vapor deposition is easy, and therefore that the abovementioned effects can be easily obtained. Preferred examples of metal oxides include molybdenum oxide, vanadium oxide and ruthenium oxide. Among the cyano group-containing compounds, more preferred are (a) a compound having at least one electron-acceptable nitrogen in addition to the nitrogen atom of a cyano group, and further having a cyano group in the molecule, (b) a compound having both a halogen and a cyano group in the molecule, (c) a compound having both a carbonyl group and a cyano group in the molecule, and (d) a compound containing all of an electron-acceptable nitrogen in addition to the nitrogen atom of a cyano group, a halogen and a cyano group, since the compounds become strong electron acceptors. In the case where the hole injection layer consists of an acceptor compound alone, or in the case where the hole injection layer is doped with an acceptor compound, the hole injection layer can be a single layer, but can also comprise multiple layers laminated. A suitable doping concentration in the case where an acceptor compound is a dopant depends on the material and the film thickness of the doped region, but preferred is 50 wt% or less, and more preferred is 10 wt% or less.

The anode is not especially limited, if the material allows holes to be efficiently injected into an organic layer, but it is preferred to use a material with a relatively large work function. As examples of the material of the anode, enumerated are conductive metal oxides such as tin oxide, indium oxide, indium zinc oxide and indium tin oxide (ITO), inorganic conductive materials such as metals like gold, silver and chromium, copper iodide and copper sulfide, conductive polymers such as polythiophenes, polypyrroles and polyanilines. Any one of these electrode materials can be used alone, and a plurality of the materials can also be used as a laminate or mixture.

The resistance of the anode is only required to be such that a current enough for light emission of the light-emitting element can be supplied, but in view of the power consumption of the light-emitting element, a low resistance is desirable. For example, if the resistance is 300 Ω/□ or less, the anode can function as an electrode. However, ITO substrates of approx. 10 Ω/□ are commercially available at present, and consequently it is especially desirable to use a low-resistance product of 100 Ω/□ or less. The thickness of the anode can be selected as desired for adaptation to the resistance value, but usually anodes of 100 to 300 nm are often used.

Further, in order to keep the mechanical strength of the light-emitting element, it is preferred to form the anode on a substrate. As the substrate, a glass substrate such as soda glass or non-alkali glass can be suitably used. The thickness of the glass substrate is only required to be a thickness enough to keep the mechanical strength, and consequently 0.5 mm or more is sufficient. As the material of the glass, it is desirable that the amount of ions dissolved from the glass is smaller, and therefore a non-alkali glass is preferred. However, a soda lime glass coated with a barrier such as SiO₂ is also commercially available, and consequently can be used. Furthermore, if the anode functions stably, the substrate is not required to be glass, and for example, the anode can also be formed on a plastic substrate. The method for forming the anode is not especially limited, and for example, an electron beam method, sputtering method, chemical reaction method or the like can be used.

The material used as the cathode is not especially limited, if the material allows electrons to be efficiently injected into an organic layer, and as examples of the material, enumerated are platinum, gold, silver, copper, iron, tin, zinc, aluminum, indium, chromium, lithium, sodium, potassium, cesium, calcium and magnesium, and alloys thereof, etc. In order to enhance the electron injection efficiency, for thereby improving element properties, lithium, sodium, potassium, cesium, calcium, magnesium and an alloy containing any of these metals with a low work function are effective. However, in general, these metals with a low work function are often unstable in atmosphere, and therefore a method for obtaining an electrode with high stability by doping the organic layer with a slight amount (1 nm or less in terms of a film thickness meter for vapor deposition) of lithium or magnesium is a preferred example. Moreover, an inorganic salt such as lithium fluoride can also be used. Further, for protecting the electrode, laminating a metal such as platinum, gold, silver, copper, iron, tin, aluminum or indium, an alloy containing any of these metals, an inorganic substance such as silica, titania or silicon nitride, or an organic polymeric compound such as polyvinyl alcohol, polyvinyl chloride or hydrocarbon-based polymeric compound is a preferred example. The method for forming the cathode is not especially limited, and for example, resistance heating, electron beam, sputtering, ion plating, coating or the like can be used.

The light-emitting layer can be provided either as a single layer or a plurality of layers, and either of them can be formed of a light-emitting material (host material, dopant material). The light-emitting material can be either a mixture comprising the host material and the dopant material or the host material alone. That is, in each light-emitting layer of the light-emitting element of this invention, either the host material alone or the dopant material alone can emit light, or both the host material and the dopant material can emit light. From the viewpoints of efficiently using the electric energy and obtaining light emission of high color purity, it is preferred that the light-emitting layer is formed of a mixture comprising the host material and the dopant material. Further, as the host material and the dopant material, one type alone each can be used, or a plurality of types each can also be used in combination. The dopant material can be contained either in the entire host material or partially in the host material. The dopant material can also be either laminated or dispersed. The light color emitted by the dopant material can be controlled. If the amount of the dopant material is too large, a concentration quenching phenomenon occurs. Consequently it is preferred to use 20 wt% or less of the dopant material on the basis of the weight of the host material. More preferred is 10 wt% or less. As the doping method, the dopant material can be vapor-deposited together with the host material, or a mixture obtained by mixing the dopant material with the host material can also be simultaneously vapor-deposited.

Specific examples of the light-emitting material include condensed ring derivatives such as anthracene and pyrene, tris(8-quinolinolate)aluminum and other metal-chelated oxinoid compounds, bisstyryl derivatives such as bisstyrylanthracene derivatives and distyrylbenzene derivatives, tetraphenylbutadiene derivatives, indene derivatives, coumarin derivatives, oxadiazole derivatives, pyrrolopyridine derivatives, perynone derivatives, cyclopendadiene derivatives, oxadiazole derivatives, thiadiazolopyridine derivatives, dibenzofuran derivatives, carbazole derivatives, indolocarbazole derivatives, and polymers such as polyphenylenevinylene derivatives, polyparaphenylene derivatives, andpolythiophene derivatives, and the like, respectively known hitherto as light-emitting materials, though not limited thereto.

The host material contained in the light-emitting material is not especially limited, but examples of it include compounds having a condensed aryl ring such as naphthalene, anthracene, phenanthrene, pyrene, chrysene, naphthacene, triphenylene, perylene, fluoranthene, fluorene and indene, derivatives thereof, aromatic amine derivatives such as N,N'-dinaphthyl-N,N'-diphenyl-4,4'-diphenyl-1,1'-diamine, tris(8-quinolinate)aluminum (III) and other metal-chelated oxinoid compounds, bisstyryl derivatives such as distyrylbenzene derivatives, tetraphenylbutadiene derivatives, indene derivatives, coumarin derivatives, oxadiazole derivatives, pyrrolopyridine derivatives, perynone derivatives, cyclopentadiene derivatives, pyrrolopyrrole derivatives, thiadiazolopyridine derivatives, dibenzofuran derivatives, carbazole derivatives, indolocarbazole derivatives, triazine derivatives, polymers such as polyphenylenevinylene derivatives, polyparaphenylene derivatives, polyfluorene derivatives, polyvinyl carbazole derivatives, polythiophene derivatives, and the like, though not especially limited thereto. Further, the dopant material is not especially limited, but examples of it include compounds having a condensed aryl ring such as naphthalene, anthracene, phenanthrene, pyrene, chrysene, triphenylene, perylene, fluoranthene, fluorene and indene, derivatives thereof (for example, 2-(benzothiazole-2-yl)-9,10-diphenylanthracene, 5,6,11,12-tetraphenylnaphthacene, etc.), compounds having a heteroaryl ring such as furan, pyrrole, thiophene, silole, 9-silafluorene, 9,9'-spirobisilafluorene, benzothiophene, benzofuran, indole,dibenzothiophene, dibenzofuran, imidazopyridine, phenanthroline, pyridine, pirazine, naphthyridine, quinoxaline, pyrrolopyridine and thioxanthene, derivatives thereof, borane derivatives, distyrylbenzene derivatives, aminostyryl derivatives such as 4,4'-bis(2-(4-diphenylaminophenyl)ethenyl)biphenyl, and 4,4'-bis(N-stilbene-4-yl)-N-phenylamino)stilbene, aromatic acetylene derivatives, tetraphenylbutadiene derivatives, stilbene derivatives, aldazine derivatives, pyrromethene derivatives, diketopyrrolo[3,4-c]pyrrole derivatives, coumarin derivatives such as 2,3,5,6-1H,4H-tetrahydro-9-(2'-benzothiazolyl)quinolidino[9,9a,1-gh]coumarin, azole derivatives such as imidazole, thiazole, thiadiazole, carbazole, oxazole, oxadiazole and triazole, metal complexes thereof, aromatic amine derivatives typified by N,N'-diphenyl-N,N'-di(3-methylphenyl)-4,4'-diphenyl-1,1'-diamine, etc.

Further, the light-emitting layer may also contain a phosphorescent light-emitting material. A phosphorescent light-emitting material is a material emitting phosphorescent light even at room temperature. The dopant is basically required to provide phosphorescent light emission even at room temperature, and is not especially limited, and an organic metal complex compound containing at least one metal selected from the group comprising iridium (Ir), ruthenium (Ru), rhodium (Rh), palladium (Pd), platinum (Pt), Osmium (Os) and rhenium (Re) is preferred. Among them, from the viewpoint of having a high phosphorescent light emission yield even at room temperature, an organic metal complexhaving iridium or platinum is more preferred. As the host of the phosphorescent light-emitting material, an aromatic hydrocarbon compound derivative such as an indole derivative, carbazole derivative, indolocarbazole derivative, nitrogen-containing aromatic compound having pyridine, pyrimidine or triazine skeleton, polyarylbenzene derivative, spirofluorene derivative, or truxene derivative, a compound containing a chalcogen element such as dibenzofuran derivative or dibenzothiophene derivative, an organic metal complex such as beryllium quinolinol complex, or the like can be suitably used. However, basically the host is not especially limited thereto, if it is larger than the dopant in triplet energy and allows smooth injection and transport of electrons and holes from the respective transport layers. Further, two or more triplet light-emitting dopants can also be contained, and two or more host materials can also be contained. Furthermore, one or more triplet light-emitting dopants and one or more fluorescent light-emitting dopants can also be contained.

Preferred phosphorescent light-emitting hosts and dopants are not especially limited, and the following specific examples can be enumerated.

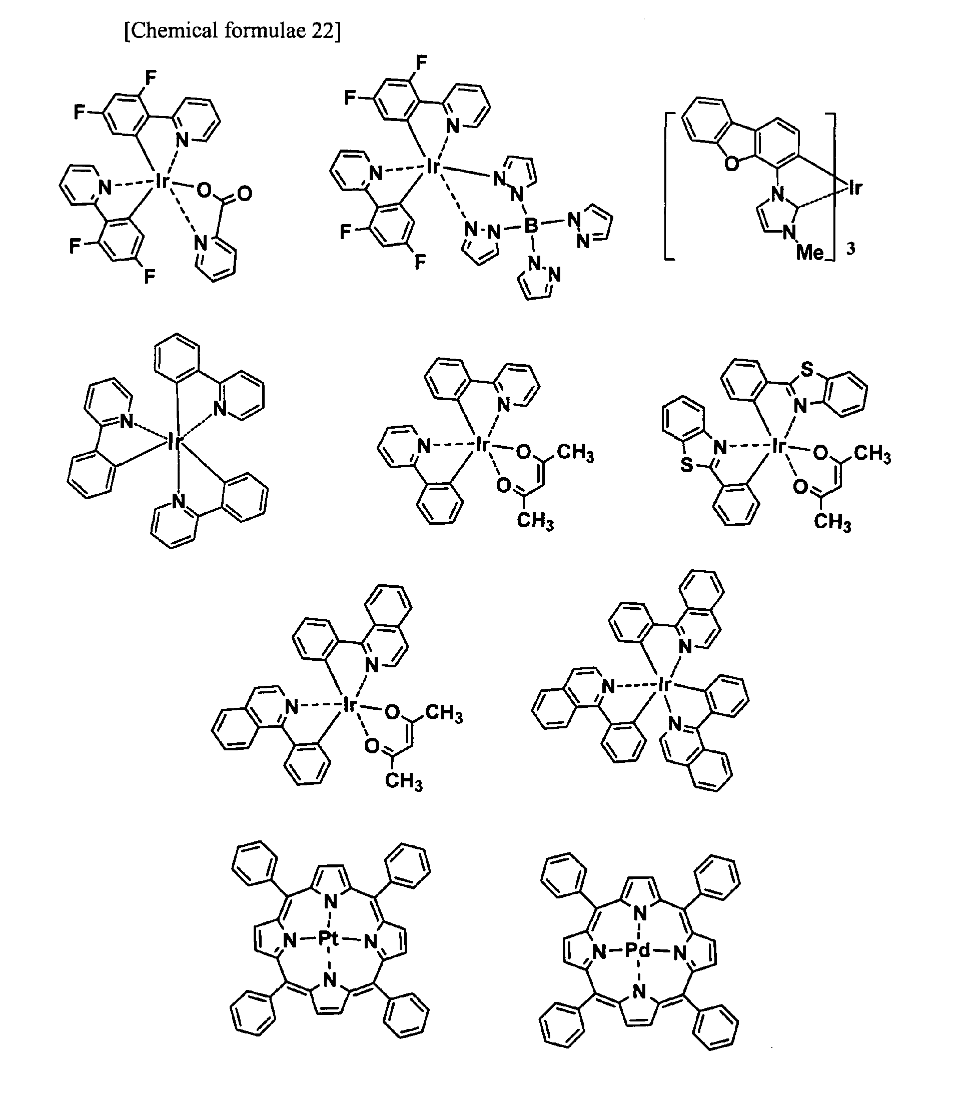

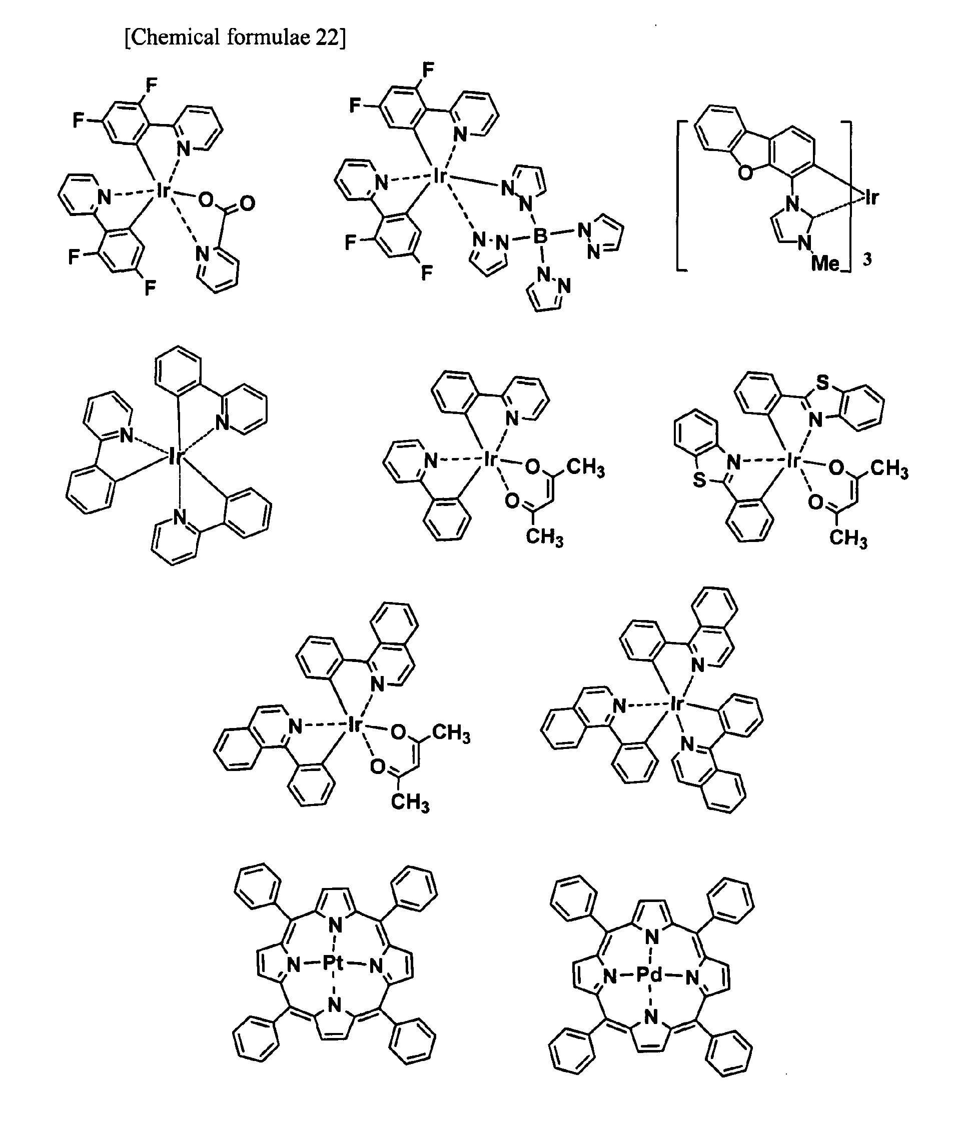

A compound represented by general formula (1) has good hole injection and transport properties and high electron-blocking performance, and in addition, has a high triplet level. Consequently in the case where a phosphorescent light-emitting layer and a hole transport layer containing a compound represented by general formula (1) are combined, the triplet energy migration from the phosphorescent light-emitting layer to the hole transport layer is inhibited, and the thermal inactivation of phosphorescent light energy in the hole transport layer can be prevented. For this reason, the decline of light-emission efficiency can be prevented, and a light-emitting element driven at a low voltage and having long life can be preferably obtained.

In this invention, between the cathode and the electron transport layer, an electron injection layer can also be formed. In general, the electron injection layer is inserted for the purpose of helping the injection of electrons from the cathode into the electron transport layer. In the case where it is inserted, the abovementioned compound having a heteroaryl ring structure containing an electron-acceptable nitrogen can be used as it is, or a layer containing the abovementioned donor compound can also be used. Further, as the electron injection layer, an inorganic substance such as an insulator or semiconductor can also be used. It is preferred to use these materials for such reasons that the short-circuit of the light-emitting element can be effectively prevented and that the electron injection capability can be enhanced. As the insulator, it is preferred to use at least one metal compound selected from the group comprising alkali metal chalcogenides, alkaline earth metal chalcogenides, alkali metal halides and alkaline earth metal halides. It is preferred that the electron injection layer comprises any of these alkali metal chalcogenides and the like, since the electron injection capability can be further enhanced. Specifically, preferred examples of the alkali metal chalcogenides include Li₂O, Na₂S and Na₂Se, and preferred examples of alkaline earth metal chalcogenides include CaO, BaO, SrO, BeO, BaS and CaSe. Further, preferred examples of alkali metal halides include LiF, NaF, KF, LiCl, KCl and NaCl, etc. Furthermore, preferred alkaline earth metal halides include fluorides such as CaF₂, BaF₂, SrF₂, MgF₂ and BeF₂ and halides other than fluorides. Moreover, a complex comprising an organic substance and a metal can also be suitably used. In the case where an inorganic substance such as an insulator or semiconductor is used as the electron injection layer, if the layer thickness is too thick, there arise such problems that the light-emitting element is insulated, and that the drive voltage becomes high. That is, the thickness margin of the electron injection layer is so narrow that the yield at the time of producing the light-emitting element may decline. However, it is more preferred that a complex comprising an organic substance and a metal is used as the electron injection layer, since the layer thickness adjustment is easy. With regard to examples of such an organic metal complex, as preferred examples of the organic substance in the complex with an organic substance, enumerated are quinolinol, benzoquinolinol, pyridyl phenol, flavonol, hydroxyimidazopyridine, hydroxybenzazole, hydroxytriazole, etc. Among them, a complex comprising an alkali metal and an organic substance is preferred, and a complex comprising lithium and an organic substance is more preferred. Lithium quinolinol is especially preferred.

The light-emitting element of this invention has a function of converting electric energy into light. In this case, as the electric energy, DC current is mainly used, but pulse current and AC current can also be used. The current value and the voltage value are not especially limited, but considering the power consumption and the life of the element, they should be selected to obtain the highest luminance at an energy as low as possible.

The light-emitting element of this invention can be suitably used, for example, in a display for displaying in matrix mode and/or segment mode.

In matrix mode, the pixels for display are disposed two-dimensionally in lattice or mosaic, and sets of pixels are used to display characters and images. The form and size of a pixel are decided in response to applications. For example, for displaying images and characters on a personal computer, monitor or television, usually a square pixel of 300 µm or less per side is used. Further, in the case of a large display such as a display panel, a pixel on the order of millimeters per side is used. In the case of monochromatic display, it is only required to dispose pixels of the same color, but in the case of color display, pixels of red, green and blue are disposed for displaying. In this case, typically delta type and stripe type are available. Further, as the matrix drive method, either the line sequential drive method or the active matrix method can be used. The line sequential drive method is simple in structure, but considering the operation characteristics, the active matrix method may be more excellent as the case may be. Consequently it is necessary to selectively use the methods in response to applications.

In the segment mode of this invention, a pattern is formed to display predetermined information, and the regions decided by the arrangement of the pattern are made to emit light. Examples of the segment mode include time display by a digital clock, temperature display by a thermometer, action state display in an audio device, electromagnetic cooker, etc. Further, both the matrix display and the segment display may also be present in the same panel.

The light-emitting element of this invention can be preferably used also for backlights of various devices, etc. A backlight is used mainly for the purpose of enhancing the visibility of a display device that does not spontaneously emit light, and is used in a liquid crystal display device, clock, audio device, vehicle panel, display panel, signboard, etc. In particular, the light-emitting element of this invention can be preferably used for backlights of liquid crystal display devices, above all, personal computers desired to be made thinner, and can provide thin and light-weight backlights compared with conventional backlights.

### EXAMPLES

This invention is explained below in reference to examples, but is not limited thereto or thereby. Meanwhile, the numbers of the compounds stated in the following examples indicate the numbers of the abovementioned compounds. Further, in Tables 1 to 7, a first electron transport layer is an electron transport layer kept in contact with a light-emitting layer, and a second electron transport layer is not kept in contact with a light-emitting layer and refers to an electron transport layer laminated further on the first electron transport layer. However, in the case where there is no first electron transport layer and where a second electron transport layer only is present as the electron transport layer, the second electron transport layer is kept in contact with the light-emitting layer.

### Example 1

A glass substrate with 165 nm of an ITO transparent conductive film deposited (produced by Geomatec Co., Ltd., 11 Ω/□, sputtered product) was cut to 38 × 46 mm, and etched. The obtained substrate was ultrasonically washed with "SEMICO CLEAN 56" (trade name, produced by Furuuchi Chemical Corporation) for 15 minutes, and washed with ultrapure water. The substrate was treated with UV-ozone for 1 hour immediately before the element was produced, and installed in a vacuum deposition apparatus. The air in the apparatus was exhausted to achieve a vacuum degree of 5 × 10⁻⁴ Pa or less in the apparatus. A resistance heating method was used to vapor-deposit compound [1] as a hole transport layer by 60 nm. Then, using compound (H-1) as a host material and compound (D-1) as a dopant material, a light-emitting layer was vapor-deposited to achieve a dopant material doping concentration of 5 wt% and to have a thickness of 40 nm. Subsequently using compound (E-1) as an electron transport material and Liq as a donor compound, a second electron transport layer was laminated to achieve a dopant concentration of 50 wt% and to have a thickness of 20 nm.

Then, as an electron injection layer, Liq was vapor-deposited by 0.5 nm, and subsequently magnesium and silver were vapor-deposited by 1000 nm to achieve a ratio by weight of 1:1 for forming a cathode, thus preparing a 5 × 5 mm square element. The layer thickness in this case was the value displayed on a crystal oscillator film thickness monitor. The light-emitting element was driven by DC at 10 mA/cm², and blue light emission was obtained at a drive voltage of 4.8 V and an external quantum efficiency of 4.1%. The durability life was measured with the initial luminance set at 1000 cd/m², and154 hours later, the luminance decreased by 20% compared with the initial luminance. Meanwhile compounds (H-1), (D-1), (E-1) and Liq are the following compounds.

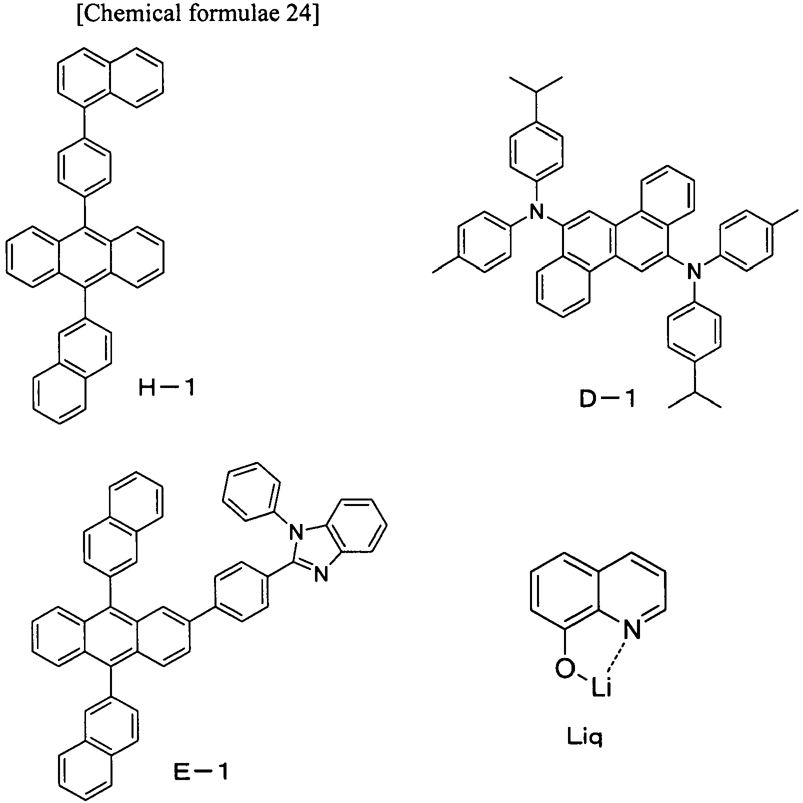

### Examples 2 to 8

Light-emitting elements were produced and evaluated as described in Example 1, except that the materials shown in Table 1 were used as a hole transport layer, light-emitting layer host material, light-emitting layer dopant material and second electron transport layer. The results of the respective examples are shown in Table 1. Meanwhile, compound (E-2) is the following compound.

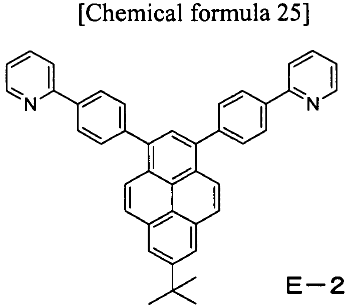

### Example 7

A glass substrate with 165 nm of an ITO transparent conductive film deposited (produced by Geomatec Co., Ltd., 11 Ω/□, sputtered product) was cut to 38 × 46 mm, and etched. The obtained substrate was ultrasonically washed with "SEMICO CLEAN 56" (trade name, produced by Furuuchi Chemical Corporation) for 15 minutes, and washed with ultrapure water. The substrate was treated with UV-ozone for 1 hour immediately before the element was produced, and installed in a vacuum deposition apparatus. The air in the apparatus was exhausted to achieve a vacuum degree of 5 × 10⁻⁴ Pa or less in the apparatus. A resistance heating method was used to vapor-deposit compound [1] as a hole transport layer by 60 nm. Then, using compound (H-1) as a host material and compound (D-1) as a dopant material, a light-emitting layer was vapor-deposited to achieve a dopant material doping concentration of 5 wt% and to have a thickness of 40 nm. Subsequently E-2was vapor-deposited as a first electron transport layer by 5 nm, and further compound (E-2) was laminated as an electron transport material of a second electron transport layer using cesium as a donor compound, to achieve a donor compound doping concentration of 20 wt% and to have a thickness of 15 nm.

Then, magnesium and silver were vapor-deposited by 1000 nm to achieve a ratio by weight of 1:1 for forming a cathode, thus preparing a 5 × 5 mm square element. The layer thickness in this case was the value displayed on a crystal oscillator film thickness monitor. The light-emitting element was driven by DC at 10 mA/cm², and blue light emission was obtained at a drive voltage of 4.6 V and an external quantum efficiency of 4.2%. The durability life was measured with the initial luminance set at 1000 cd/m², and 130 hours later, the luminance decreased by 20% compared with the initial luminance.

### Examples 8 and 9

Light-emitting elements were produced and evaluated as described in Example 7, except that the materials stated in Table 1 were used as a hole transport layer, a light-emitting layer host material, a light-emitting layer dopant material, a first electron transport layer and a second electron transport layer. The results of the respective examples are shown in Table 1.

### Examples 10 to 12

Light-emitting elements were produced and evaluated as described in Example 1, except that the compound stated in Table 1 was used as a hole transport layer, that H-2 was used as a light-emitting layer host material, that D-2 was used as a light-emitting layer dopant material, and that the dopant concentration was 10 wt%. Meanwhile, the durability life was evaluated by setting the initial luminance at 4000 cd/m². The results of the respective examples are shown in Table 1. Further, H-2 and D-2 are the following compounds.

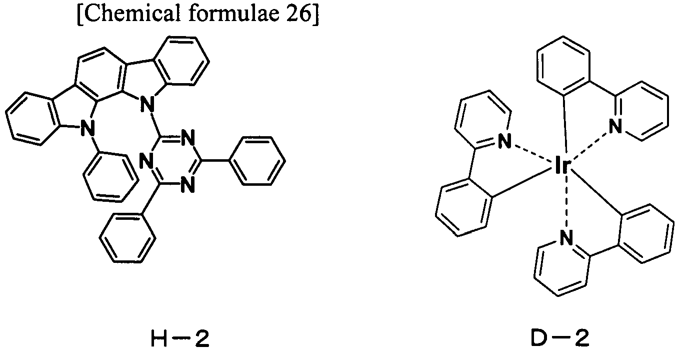

### Examples 13 to 15

Light-emitting elements were produced and evaluated as described in Example 7, except that the compound stated in Table 1 was used as a hole transport layer, that H-2 was used as a light-emitting layer host material, that D-2 was used as a light-emitting layer dopant material, and that the dopant material concentration was 10 wt%. The results of the respective examples were shown in Table 1. Meanwhile, the durability life was evaluated by setting the initial luminance at 4000 cd/m².

### Comparative Example 1

A glass substrate with 165 nm of an ITO transparent conductive film deposited (produced by Geomatec Co., Ltd., 11 Ω/□, sputtered product) was cut to 38 × 46 mm, and etched. The obtained substrate was ultrasonically washed with "SEMICO CLEAN 56" (trade name, produced by Furuuchi Chemical Corporation) for 15 minutes, and washed with ultrapure water. The substrate was treated with UV-ozone for 1 hour immediately before the element was produced, and installed in a vacuum deposition apparatus. The air in the apparatus was exhausted to achieve a vacuum degree of 5 × 10⁻⁴ Pa or less in the apparatus. A resistance heating method was used to vapor-deposit compound [1] as a hole transport layer by 60 nm. Then, using compound (H-1) as a host material and compound (D-1) as a dopant material, a light-emitting layer was vapor-deposited to achieve a dopant material doping concentration of 5 wt% and to have a thickness of 40 nm. Subsequently compound (E-1) was vapor-deposited as an electron transport material to form a second electron transport layer with a thickness of 20 nm.

Then, as an electron injection layer, Liq was vapor-deposited by 0.5 nm, and subsequently magnesium and silver were vapor-deposited by 1000 nm to achieve a ratio by weight of 1:1 for forming a cathode, thus preparing a 5 × 5 mm square element. The layer thickness in this case was the value displayed on a crystal oscillator film thickness monitor. The light-emitting element was driven by DC at 10 mA/cm², and blue light emission was obtained at a drive voltage of 6.0 V and an external quantum efficiency of 3.2%. The durability life was measured by setting the initial luminance at 1000 cd/m², and 84 hours later, the luminance decreased by 20% compared with the initial luminance.

### Comparative Examples 2 to 6

Light-emitting elements were produced and evaluated as described in Comparative Example 1, except that the materials stated in Table 2 were used as a hole transport layer, a light-emitting layer host material, a light-emitting layer dopant material and a second electron transport layer. The results of the respective comparative examples are shown in Table 2.

### Comparative Examples 7 to 12

Light-emitting elements were produced and evaluated as described in Example 1, except that the materials stated in Table 2 were used as a hole transport layer, a light-emitting layer host material, a light-emitting layer dopant material and a second electron transport layer. The results of the respective comparative examples are shown in Table 2. Meanwhile, HT-1, HT-2 and HT-3 are the following compounds.

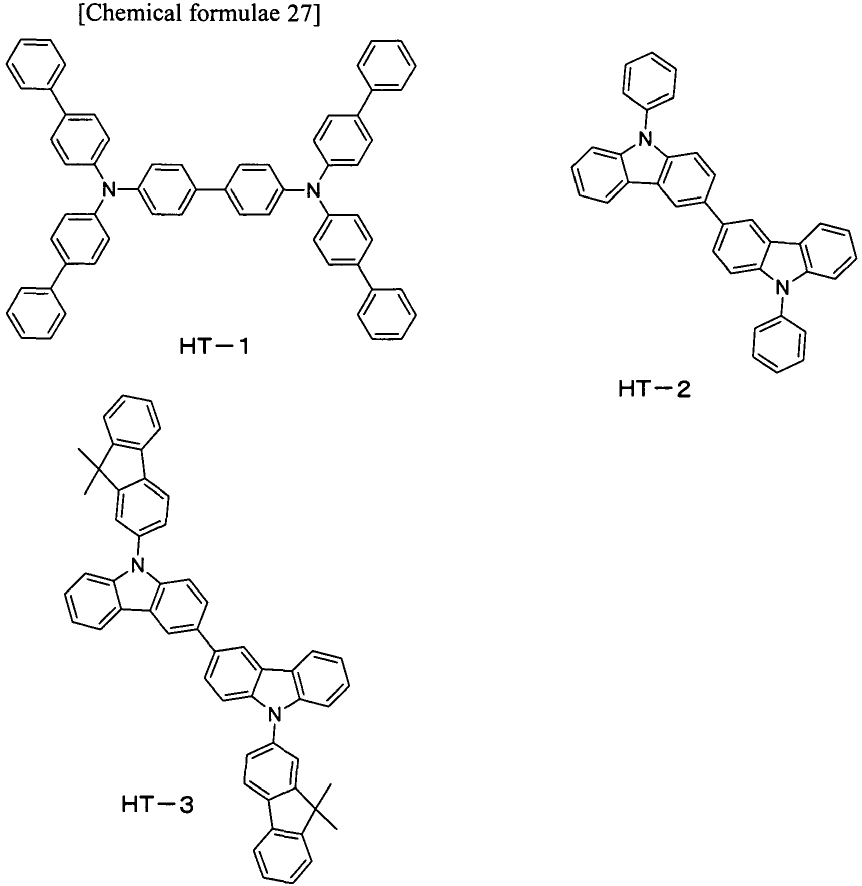

### Comparative Examples 13 to 15

Light-emitting elements were produced and evaluated as described in Example 7, except that the materials stated in Table 2 were used as a hole transport layer, a light-emitting layer host material, a light-emitting layer dopant material, a first electron transport layer and a second electron transport layer. The results are shown in Table 2.

### Comparative Examples 16 to 18

Light-emitting elements were produced as described in Example 10, except that the compound stated in Table 2 was used as a hole transport layer, H-2 is used as a light-emitting layer host material and D-2 was used as a light-transmitting layer dopant material. Meanwhile, the durability life was evaluated by setting the initial luminance at 4000 cd/m². The results of the respective comparative examples are shown in Table 2.

### Comparative Examples 19 to 21

Light-emitting elements were produced and evaluated as described in Example 13, except that the compound stated in Table 2 was used as a hole transport layer, that H-2 was used as a light-emitting layer host material, and that D-2 was used as a light-emitting layer dopant material. Meanwhile, the durability life was evaluated by setting the initial luminance at 4000 cd/m². The results of the respective comparative examples are shown in Table 2.

### Example 16

A glass substrate with 165 nm of an ITO transparent conductive film deposited (produced by Geomatec Co., Ltd., 11 Ω/□, sputtered product) was cut to 38 × 46 mm, and etched. The obtained substrate was ultrasonically washed with "SEMICO CLEAN 56" (trade name, produced by Furuuchi Chemical Corporation) for 15 minutes, and °washed with ultrapure water. The substrate was treated with UV-ozone for 1 hour immediately before the element was produced, and installed in a vacuum deposition apparatus. The air in the apparatus was exhausted to achieve a vacuum degree of 5 × 10⁻⁴ Pa or less in the apparatus. A resistance heating method was used to vapor-deposit, at first, an acceptor compound, HAT-CN6 by 10 nm as a hole injection layer, and then, compound [1] by 50 nm as a hole transport layer. Then, using compound (H-1) as a host material and compound (D-1) as a dopant material, a light-emitting layer was vapor-deposited to achieve a dopant material doping concentration of 5 wt% and to have a thickness of 40 nm. Subsequently an electron transport material, compound (E-1) was laminated as a second electron transport layer, using Liq as a donor compound, to achieve a donor compound doping concentration of 50 wt% and to have a thickness of 20 nm.

Then, as an electron injection layer, Liq was vapor-deposited by 0.5 nm, and subsequently magnesium and silver were vapor-deposited by 1000 nm to achieve a ratio by weight of 1:1 for forming a cathode, thus preparing a 5 × 5 mm square element. The layer thickness in this case was the value displayed on a crystal oscillator film thickness monitor. The light-emitting element was driven by DC at 10 mA/cm², and blue light emission was obtained at a drive voltage of 4.0 V and an external quantum efficiency of 5.5%. The durability life was measured by setting the initial luminance at 1000 cd/m², and 361 hours later, the luminance decreased by 20% compared with the initial luminance. Meanwhile, HAT-CN6 is the following compound.

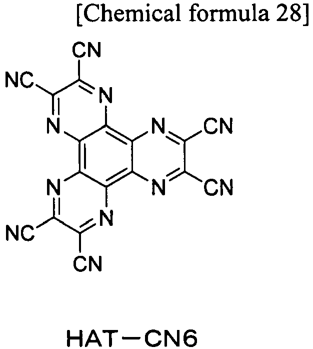

### Examples 17 and 18

Light-emitting elements were produced and evaluated as described in Example 16, except that the compound stated in Table 3 was used as a hole transport layer. The results of the respective examples are shown in Table 3.

### Example 19

A glass substrate with 165 nm of an ITO transparent conductive film deposited (produced by Geomatec Co., Ltd., 11 Ω/□, sputtered product) was cut to 38 × 46 mm, and etched. The obtained substrate was ultrasonically washed with "SEMICO CLEAN 56" (trade name, produced by Furuuchi Chemical Corporation) for 15 minutes, and washed with ultrapure water. The substrate was treated with UV-ozone for 1 hour immediately before the element was produced, and installed in a vacuum deposition apparatus. The air in the apparatus was exhausted to achieve a vacuum degree of 5 × 10⁻⁴ Pa or less in the apparatus. A resistance heating method was used to vapor-deposit, at first, a hole injection material, HT-4 as the hole injection layer, using an acceptor compound, F4-TCNQ, to achieve an acceptor compound doping concentration of 10 wt% and to have a thickness of 30 nm. Then, compound [1] was vapor-deposited as a hole transport layer by 30 nm. Subsequently using compound (H-1) as a host material and compound (D-1) as a dopant material, a light-emitting layer was vapor-deposited to achieve a dopant material doping concentration of 5 wt% and to have a thickness of 40 nm. Then, an electron transport material, compound (E-1) was laminated as a second electron transport layer, using Liq as a donor compound, to achieve a donor compound doping concentration of 50 wt% and to have a thickness of 20 nm.

Subsequently Liq was vapor-deposited as an electron injection layer by 0.5 nm, and then magnesium and silver were vapor-deposited by 1000 nm to achieve a ratio by weight of 1:1 for forming a cathode, thus producing a 5 × 5 square element. The layer thickness in this case was the value displayed on a crystal oscillator film thickness monitor. The light-emitting element was driven by DC at 10 mA/cm², and blue light emission was obtained at a drive voltage of 4.1 V and an external quantum efficiency of 5.3%. The durability life was measured by setting the initial luminance at 1000 cd/m², and 368 hours later, the luminance decreased by 20% compared with the initial luminance. Meanwhile, HT-4 and F4-TCNQ are the following compounds.

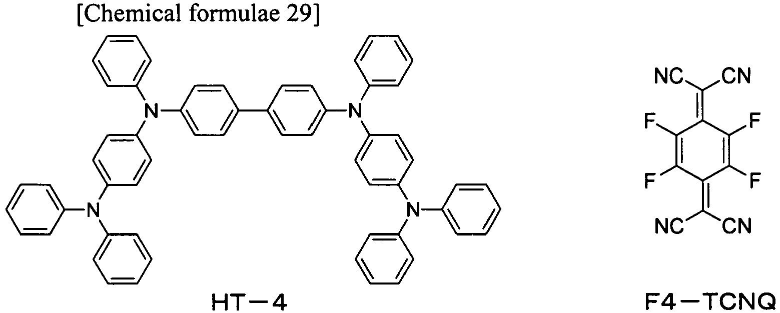

### Examples 20 and 21

Light-emitting elements were produced and evaluated as described in Example 19, except that the compound stated in Table 3 was used as a hole transport layer. The results of the respective examples are shown in Table 3.

### Examples 22 to 24

Light-emitting elements were produced and evaluated as described in Example 16, except that the materials stated in Table 3 were used as a hole injection layer, a hole transport layer, a emitting-light layer host material, a light emitting-light layer dopant material and a second electron transport layer. The results of the respective examples are shown in Table 3.

### Examples 25 to 27

Light-emitting elements were produced and evaluated as described in Example 19, except that the materials stated in Table 3 were used as a hole injection layer, a hole transport layer, a light-emitting layer host material, a light-emitting layer dopant material and a second electron transport layer. The results of the respective examples are shown in Table 3.

### Examples 28 to 33

Light-emitting elements were produced and evaluated as described in Example 16, except that the materials stated in Table 3 were used as a hole injection layer, a hole transport layer, a light-emitting layer host material, a light-emitting layer dopant material and a second electron transport layer. The results of the respective examples are shown in Table 3. Meanwhile, E-3 and E-4 are the following compounds.

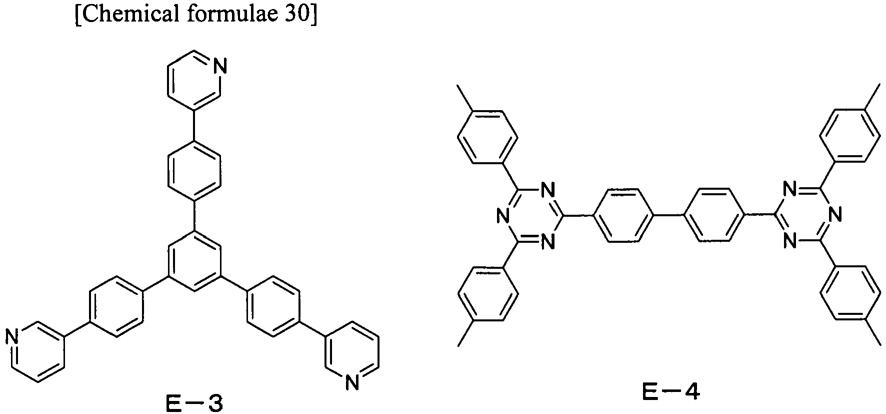

### Example 34

A glass substrate with 165 nm of an ITO transparent conductive film deposited (produced by Geomatec Co., Ltd., 11 Ω/□, sputtered product) was cut to 38 × 46 mm, and etched. The obtained substrate was ultrasonically washed with "SEMICO CLEAN 56" (trade name, produced by Furuuchi Chemical Corporation) for 15 minutes, and washed with ultrapure water. The substrate was treated with UV-ozone for 1 hour immediately before the element was produced, and installed in a vacuum deposition apparatus. The air in the apparatus was exhausted to achieve a vacuum degree of 5 × 10⁻⁴ Pa or less in the apparatus. A resistance heating method was used to, at first, vapor-deposit HAT-CN6 as a hole injection layer by 10 nm, and then to vapor-deposit compound [1] as a hole transport layer by 50 nm. Subsequently, using compound (H-1) as a host material and compound (D-1) as a dopant material, a light-emitting layer was vapor-deposited to achieve a dopant material doping concentration of 5 wt% and to have a thickness of 40 nm. Then as a first electron transport layer, E-2 was vapor-deposited by 5 nm, and further an electron transport material, compound (E-2) was laminated as a second electron transport layer, using cesium as a donor compound, to achieve a donor compound doping concentration of 20 wt% and to have a thickness of 15 nm.

Subsequently, magnesium and silver were vapor-deposited by 1000 nm to achieve a ratio by weight of 1:1 for forming a cathode, thus preparing a 5 × 5 mm square element. The layer thickness in this case was the value displayed on a crystal oscillator film thickness monitor. The light-emitting element was driven by DC at 10 mA/cm², and blue light emission was obtained at a drive voltage of 3.7 V and an external quantum efficiency of 5.2%. The durability life was measured by setting the initial luminance at 1000 cd/m², and 345 hours later, the luminance decreased by 20% compared with the initial luminance.

### Examples 35 and 36

Light-emitting elements were produced and evaluated as described in Example 34, except that the compound stated in Table 3 was used as a hole transport layer. The results of the respective examples are shown in Table 3.

### Example 37

A glass substrate with 165 nm of an ITO transparent conductive film deposited (produced by Geomatec Co., Ltd., 11 Ω/□, sputtered product) was cut to 38 × 46 mm, and etched. The obtained substrate was ultrasonically washed with "SEMICO CLEAN 56" (trade name, produced by Furuuchi Chemical Corporation) for 15 minutes, and washed with ultrapure water. The substrate was treated with UV-ozone for 1 hour immediately before the element was produced, and installed in a vacuum deposition apparatus. The air in the apparatus was exhausted to achieve a vacuum degree of 5 × 10⁻⁴ Pa or less in the apparatus. A resistance heating method was used to, at first, vapor-deposit a hole injection material, HT-4 as a hole injection layer, using F4-TCNQ as an acceptor compound, to achieve an acceptor compound doping concentration of 10 wt% by 30 nm. Then, as a hole transport layer, compound [1] was vapor-deposited by 30 nm. Subsequently using compound (H-1) as a host material and compound (D-1) as a dopant material, a light-emitting layer was vapor-deposited to achieve a dopant material doping concentration of 5 wt% and to have a thickness of 40 nm. Then, E-2 was vapor-deposited as a first electron transport layer, and further an electron transport material, compound (E-2) was laminated as a second electron transport layer, using cesium as a donor compound, to achieve a donor compound doping concentration of 20 wt% and to have a thickness of 15 nm.

Subsequently, magnesium and silver were vapor-deposited by 1000 nm to achieve a ratio by weight of 1:1 for forming a cathode, thus preparing a 5 × 5 mm square element. The layer thickness in this case was the value displayed on a crystal oscillator film thickness monitor. The light-emitting element was driven by DC at 10 mA/cm², and blue light emission was obtained at a drive voltage of 3.8 V and an external quantum efficiency of 5.2%. The durability life was measured by setting the initial luminance at 1000 cd/m², and 346 hours later, the luminance decreased by 20% compared with the initial luminance.

### Examples 38 and 39

Light-emitting elements were produced and evaluated as described in Example 37, except that the compound stated in Table 3 was used as a hole transport layer. The results of the respective examples are shown in Table 3.

### Examples 40 to 48

Light-emitting elements were produced and evaluated as described in Example 16, except that the materials stated in Table 4 were used as a hole injection layer, a hole transport layer, a light-emitting layer host material, a light-emitting layer dopant material and a second electron transport layer. Meanwhile, the durability life was evaluated by setting the initial luminance at 4000 cd/m². The results of the respective examples are shown in Table 4.

### Examples 49 to 51

Light-emitting elements were produced and evaluated as described in Example 19, except that the materials stated in Table 4 were used as a hole injection layer, a hole transport layer, a light-emitting layer host material, a light-emitting layer dopant material and a second electron transport layer. Meanwhile, the durability life was evaluated by setting the initial luminance at 4000 cd/m². The results of the respective examples are shown in Table 4.

### Comparative Examples 22 to 24

Light-emitting elements were produced and evaluated as described in Example 16, except that the materials stated in Table 5 were used as a hole injection layer, a hole transport layer, a light-emitting layer host material, a light-emitting layer dopant material and a second electron transport layer. The results of the respective comparative examples are shown in Table 5.

### Comparative Examples 25 to 27

Light-emitting elements were produced and evaluated as described in Example 19, except that the materials stated in Table 5 were used as a hole injection layer, a hole transport layer, a light-emitting layer host material, a light-emitting layer dopant material and a second electron transport layer. The results of the respective comparative examples are shown in Table 5.

### Comparative Examples 28 to 30

Light-emitting elements were produced and evaluated as described in Example 22, except that the materials stated in Table 5 were used as a hole injection layer, a hole transport layer, a light-emitting layer host material, a light-emitting layer dopant material and a second electron transport layer. The results of the respective comparative examples are shown in Table 5.

### Comparative Examples 31 to 33

Light-emitting elements were produced and evaluated as described in Example 25, except that the materials stated in Table 5 were used as a hole injection layer, a hole transport layer, a light-emitting layer host material, a light-emitting layer dopant material and a second electron transport layer. The results of the respective comparative examples are shown in Table 5.

### Comparative Examples 34 to 39

Light-emitting elements were produced and evaluated as described in Example 28, except that the materials stated in Table 5 were used as a hole injection layer, a hole transport layer, a light-emitting layer host material, a light-emitting layer dopant material and a second electron transport layer. The results of the respective comparative examples are shown in Table 5.

### Comparative Examples 40 to 42

Light-emitting elements were produced and evaluated as described in Example 34, except that the materials stated in Table 5 were used as a hole injection layer, a hole transport layer, a light-emitting layer host material, a light-emitting layer dopant material, a first electron transport layer and a second electron transport layer. The results of the respective comparative examples are shown in Table 5.

### Comparative Example 43 to 45

Light-emitting elements were produced and evaluated as described in Example 37, except that the materials stated in Table 5 were used as a hole injection layer, a hole transport layer, a light-emitting layer host material, a light-emitting layer dopant material, a first electron transport layer and a second electron transport layer. The results of the respective comparative examples are shown in Table 5.

### Comparative Examples 46 to 54

Light-emitting elements were produced and evaluated as described in Example 40, except that the materials stated in Table 6 were used as a hole injection layer, a hole transport layer, a light-emitting layer host material, a light-emitting layer dopant material and a second electron transport layer. Meanwhile, the durability life was evaluated by setting the initial luminance at 4000 cd/m². The results of the respective comparative examples are shown in Table 6.

### Comparative Examples 55 to 57

Light-emitting elements were produced and evaluated as described in Example 49, except that the materials stated in Table 6 were used as a hole injection layer, a hole transport layer, a light-emitting layer host material, a light-emitting layer dopant material and a second electron transport layer. Meanwhile, the durability life was evaluated by setting the initial luminance at 4000 cd/m². The results of the respective comparative examples are shown in Table 6.

### Example 52

A glass substrate with 50 nm of an ITO transparent conductive film deposited (produced by Geomatec Co., Ltd., 11 Ω/□, sputtered product) was cut to 38 × 46 mm, and etched. The obtained substrate was ultrasonically washed with "SEMICO CLEAN 56" (trade name, produced by Furuuchi Chemical Corporation) for 15 minutes, and washed with ultrapure water. The substrate was treated with UV-ozone for 1 hour immediately before the element was produced, and installed in a vacuum deposition apparatus. The air in the apparatus was exhausted to achieve a vacuum degree of 5 × 10⁻⁴ Pa or less in the apparatus. A resistance heating method was used to, at first, vapor-deposit HAT-CN6 as a hole injection layer by 10 nm, and then to vapor-deposit compound [2] as a hole transport layer by 120 nm. Subsequently, using compound (H-3) as a host material and compound (D-3) as a dopant material, a light-emitting layer was vapor-deposited to achieve a dopant material doping concentration of 10 wt% and to have a thickness of 40 nm. Then, E-5 was vapor-deposited as a first electron transport layer by 5 nm, and further, an electron transport material, compound (E-5) was laminated as a second electron transport layer, using cesium as a donor compound, to achieve a donor compound doping concentration of 20 wt% and to have a thickness of 15 nm.

Subsequently, magnesium and silver were vapor-deposited by 1000 nm to achieve a ratio by weight of 1:1 for forming a cathode, thus preparing a 5 × 5 mm square element. The layer thickness in this case was the value displayed on a crystal oscillator film thickness monitor. The light-emitting element was driven by DC at 10 mA/cm², and red light emission was obtained at a drive voltage of 5.0 V and an external quantum efficiency of 10.2%. The durability life was measured by setting the initial luminance at 1000 cd/m², and 620 hours later, the luminance decreased by 20% compared with the initial luminance.

Meanwhile, H-3, D-3 and E-5 are the following compounds.

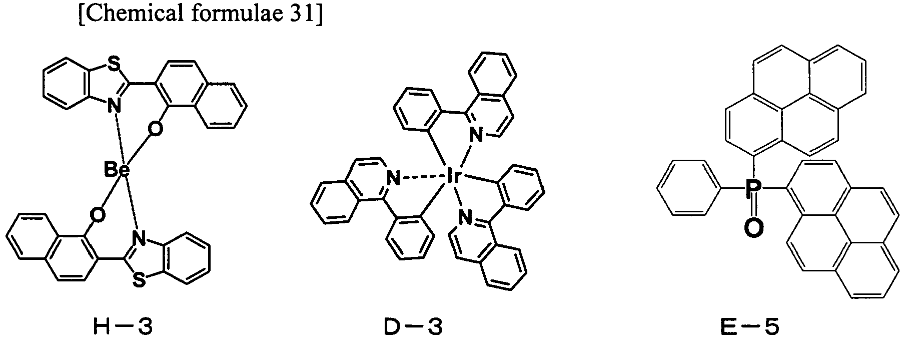

### Example 53

A light-emitting element was produced and evaluated as described in Example 52, except that a hole injection material, HT-4 was vapor-deposited as a hole injection layer by 30 nm, using A-1 as an acceptor compound, to achieve of an acceptor compound doping concentration of 10 wt%, and that compound [2] was vapor-deposited as a hole transport layer by 100 nm. The result is shown in Table 7. Meanwhile, A-1 is the following compound.

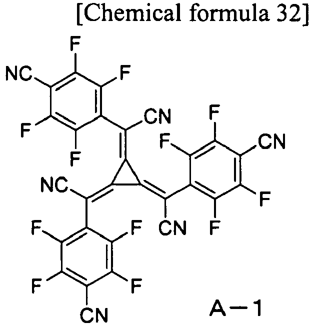

### Example 54

A light-emitting element was produced and evaluated as described in Example 52, except that E-6 was vapor-deposited as a first electrode transport layer by 5 nm, and that an electron transport material, E-6 was vapor deposited as a second electron transport layer using cesium carbonate as a donor compound, to achieve a donor compound doping concentration of 2 wt% by 15 nm. The results are shown in Table 7. Meanwhile, E-6 is the following compound.

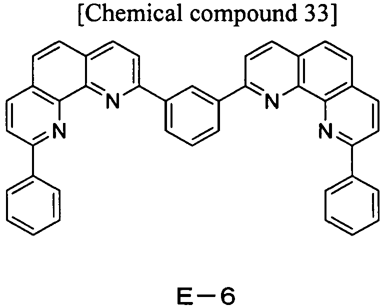

### Example 55

A light-emitting element was produced and evaluated as described in Example 53, except that E-6 was vapor-deposited as a first electron transport layer by 5 nm, and that an electron transport material, E-6 was vapor-deposited as a second electron transport layer using cesium carbonate as a donor compound, to achieve a donor compound doping concentration of 2 wt% by 15 nm. The results are shown in Table 7.

### Comparative Example 58

A light-emitting element was produced and evaluated as described in Example 52, except that HT-2 was used as a hole transport layer. The results are shown in Table 7.

### Comparative Example 59

A light-emitting element was produced and evaluated as described in Example 53, except that HT-2 was used as a hole transport layer. The results are shown in Table 7.

### Comparative Example 60

A light-emitting element was produced and evaluated as described in Example 54, except that HT-2 was used as a hole transport layer. The results are shown in Table 7.

### Comparative Example 61

A light-emitting element was produced and evaluated as described in Example 55, except that HT-2 was used as a hole transport layer. The results are shown in Table 7.

**[Table 1]**

| | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|
| | | Host material | Dopant material | Emitted light color | | Donor compound | Electron transport material | | | |
| Example 1 | [1] | H-1 | D-1 | Blue | Nil | Liq | E-1 | 4.1 | 4.8 | 154 |
| Example 2 | [12] | | | | | | | 4.1 | 4.8 | 155 |
| Example 3 | [33] | | | | | | | 4.2 | 4.8 | 151 |
| Example 4 | [1] | H-1 | D-1 | Blue | Nil | Liq | E-2 | 4.1 | 4.6 | 157 |
| Example 5 | [12] | | | | | | | 4.2 | 4.6 | 154 |
| Example 6 | [33] | | | | | | | 4.1 | 4.6 | 155 |
| Example 7 | [1] | H-1 | D-1 | Blue | E-2 | Cesium | E-2 | 4.2 | 4.6 | 130 |
| Example 8 | [12] | | | | | | | 4.1 | 4.5 | 128 |
| Example 9 | [33] | | | | | | | 4.2 | 4.6 | 124 |
| Example 10 | [1] | H-2 | D-2 | Green | Nil | Liq | E-1 | 14.5 | 4.6 | 264 |
| Example 11 | [12] | | | | | | | 14.4 | 4.6 | 257 |
| Example 12 | [33] | | | | | | | 14.4 | 4.6 | 262 |
| Example 13 | [1] | H-2 | D-2 | Green | E-2 | Cesium | E-2 | 13.2 | 4.6 | 231 |
| Example 14 | [12] | | | | | | | 13.3 | 4.5 | 238 |
| Example 15 | [33] | | | | | | | 13.3 | 4.6 | 240 |

**[Table 2-1]**

| | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|
| | | Host material | Dopant material | Emitted light color | | Donor compound | Electron transport material | | | |
| Comparative Example 1 | [1] | H-1 | D-1 | Blue | Nil | Nil | E-1 | 3.2 | 6.0 | 84 |
| Comparative Example 2 | [12] | | | | | | | 3.1 | 6.0 | 82 |
| Comparative Example 3 | [33] | | | | | | | 3.1 | 6.0 | 84 |
| Comparative Example 4 | [1] | H-1 | D-1 | Blue | Nil | Nil | E-2 | 3.2 | 5.9 | 76 |
| Comparative Example 5 | [12] | | | | | | | 3.2 | 5.9 | 72 |
| Comparative Example 6 | [33] | | | | | | | 3.1 | 5.9 | 74 |
| Comparative Example 7 | [HT-1] | H-1 | D-1 | Blue | Nil | Liq | E-1 | 3.5 | 4.8 | 82 |
| Comparative Example 8 | [HT-2] | | | | | | | 3.2 | 5.2 | 40 |
| Comparative Example 9 | [HT-3] | | | | | | | 3.1 | 5.2 | 55 |
| Comparative Example 10 | [HT-1] | H-1 | D-1 | Blue | Nil | Liq | E-2 | 3.5 | 4.7 | 79 |
| Comparative Example 11 | [HT-2] | | | | | | | 3.2 | 5.1 | 36 |
| Comparative Example 12 | [HT-3] | | | | | | | 3.2 | 5.1 | 50 |

**[Table 2-2]**

| | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|
| | | Host material | Dopant material | Emitted light color | | Donor compound | Electron transport material | | | |
| Comparative Example 13 | [HT-1] | H-1 | D-1 | Blue | E-2 | Cesium | E-2 | 3.5 | 4.6 | 61 |
| Comparative Example 14 | [HT-2] | | | | | | | 3.1 | 5.0 | 26 |
| Comparative Example 15 | [HT-3] | | | | | | | 3.0 | 5.0 | 34 |
| Comparative Example 16 | [HT-1] | H-2 | D-2 | Green | Nil | Liq | E-1 | 11.1 | 4.6 | 130 |
| Comparative Example 17 | [HT-2] | | | | | | | 13.4 | 5.1 | 70 |
| Comparative Example 18 | [HT-3] | | | | | | | 13.4 | 5.1 | 84 |
| Comparative Example 19 | [HT-1] | H-2 | D-2 | Green | E-2 | Cesium | E-2 | 10.2 | 4.6 | 120 |
| Comparative Example 20 | [HT-2] | | | | | | | 12.5 | 5.1 | 68 |
| Comparative Example 21 | [HT-3] | | | | | | | 12.3 | 5.1 | 74 |

**[Table 3-1]**

| | Hole injection layer | | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|---|---|
| | Acceptor compound | Hole injection material | | Host material | Dopant material | Emitted fight color | | Donor compound | Electron transport material | | | |
| Example 16 | HAT-CN6 | Nil | [1] | H-1 | D-1 | Blue | Nil | Liq | E-1 | 5.5 | 4.0 | 361 |
| Example 17 | | | [12] | | | | | | | 5.4 | 4.0 | 354 |
| Example 18 | | | [33] | | | | | | | 5.5 | 4.0 | 350 |
| Example 19 | F4-TCNQ | HT-4 | [1] | H-1 | D-1 | Blue | Nil | Liq | E-1 | 5.3 | 4.1 | 368 |
| Example 20 | | | [12] | | | | | | | 5.4 | 4.2 | 371 |
| Example 21 | | | [33] | | | | | | | 5.4 | 4.1 | 357 |
| Example 22 | HAT-CN6 | Nil | [1] | H-1 | D-1 | Blue | Nil | Liq | E-2 | 5.4 | 3.8 | 368 |
| Example 23 | | | [12] | | | | | | | 5.3 | 3.8 | 364 |
| Example 24 | | | [33] | | | | | | | 5.4 | 3.8 | 356 |
| Example 25 | F4-TCNQ | HT-4 | [1] | H-1 | D-1 | Blue | Nil | Liq | E-2 | 5.5 | 3.9 | 352 |
| Example 26 | | | [12] | | | | | | | 5.5 | 3.9 | 341 |
| Example 27 | | | [33] | | | | | | | 5.4 | 3.9 | 359 |

**[Table 3-2]**

| | Hole injection layer | | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|---|---|
| | Acceptor compound | Hole injection material | | Host material | Dopant material | Emitted light color | | Donor compound | Electron transport material | | | |
| Example 28 | HAT-CN6 | Nil | [1] | H-1 | D-1 | Blue | Nil | Liq | E-3 | 5.4 | 4.3 | 260 |
| Example 29 | | | [12] | | | | | | | 5.4 | 4.3 | 250 |
| Example 30 | | | [33] | | | | | | | 5.3 | 4.3 | 254 |
| Example 31 | HAT-CN6 | Nil | [1] | H-1 | D-1 | Blue | Nil | Liq | E-4 | 5.4 | 4.3 | 251 |
| Example 32 | | | [12] | | | | | | | 5.5 | 4.3 | 240 |
| Example 33 | | | [33] | | | | | | | 5.5 | 4.3 | 233 |
| Example 34 | HAT-CN6 | Nil | [1] | H-1 | D-1 | Blue | E-2 | Cesium | E-2 | 5.2 | 3.7 | 345 |
| Example 35 | | | [12] | | | | | | | 5.2 | 3.7 | 332 |
| Example 36 | | | [33] | | | | | | | 5.1 | 3.7 | 335 |
| Example 37 | F4-TCNQ | HT-4 | [1] | H-1 | D-1 | Blue | E-2 | Cesium | E-2 | 5.2 | 3.8 | 346 |
| Example 38 | | | [12] | | | | | | | 5.2 | 3.8 | 351 |
| Example 39 | | | [33] | | | | | | | 5.2 | 3.8 | 321 |

**[Table 4]**

| | Hole injection layer | | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|---|---|
| | Acceptor compound | Hole injection material | | Host material | Dopant material | Emitted light color | | Donor compound | Electron transport material | | | |
| Example 40 | HAT-CN6 | Nil | [1] | H-2 | D-2 | Green | Nil | Liq | E-1 | 18.2 | 3.9 | 592 |
| Example 41 | | | [12] | | | | | | | 18.3 | 3.9 | 575 |
| Example 42 | | | [33] | | | | | | | 18.2 | 3.8 | 572 |
| Example 43 | HAT-CN6 | Nil | [1] | H-2 | D-2 | Green | Nil | Liq | E-2 | 18.1 | 3.7 | 584 |
| Example 44 | | | [12] | | | | | | | 18.3 | 3.7 | 571 |
| Example 45 | | | [33] | | | | | | | 18.3 | 3.7 | 580 |
| Example 46 | HAT-CN6 | Nil | [1] | H-2 | D-2 | Green | Nil | Liq | E-3 | 18.1 | 4.1 | 386 |
| Example 47 | | | [12] | | | | | | | 18.2 | 4.1 | 384 |
| Example 48 | | | [33] | | | | | | | 18.0 | 4.1 | 370 |
| Example 49 | F4-TCNQ | [1] | [1] | H-2 | D-2 | Green | Nil | Liq | E-1 | 18.1 | 3.8 | 586 |
| Example 50 | | | [12] | | | | | | | 18.1 | 3.8 | 571 |
| Example 51 | | | [33] | | | | | | | 18.2 | 3.8 | 565 |

**[Table 5-1]**

| | Hole injection layer | | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|---|---|
| | Acceptor compound | Hole injection material | | Host material | Dopant material | Emitted light color | | Donor compound | Electron transport material | | | |
| Comparative Example 22 | HAT-CN6 | Nil | [HT-1] | H-1 | D-1 | Blue | Nil | Liq | E-1 | 4.9 | 4.0 | 124 |
| Comparative Example 23 | | | [HT-2] | | | | | | | 4.6 | 4.4 | 74 |
| Comparative Example 24 | | | [HT-3] | | | | | | | 4.6 | 4.4 | 84 |
| Comparative Example 25 | F4-TCNQ | HT-4 | [HT-1] | H-1 | D-1 | Blue | Nil | Liq | E-1 | 4.8 | 4.0 | 133 |
| Comparative Example 26 | | | [HT-2] | | | | | | | 4.5 | 4.4 | 72 |
| Comparative Example 27 | | | [HT-3] | | | | | | | 4.5 | 4.4 | 94 |
| Comparative Example 28 | HAT-CN6 | Nil | [HT-1] | H-1 | D-1 | Blue | Nil | Liq | E-2 | 4.8 | 3.8 | 110 |
| Comparative Example 29 | | | [HT-2] | | | | | | | 4.4 | 4.2 | 70 |
| Comparative Example 30 | | | [HT-3] | | | | | | | 4.5 | 4.2 | 80 |
| Comparative Example 31 | F4-TCNQ | HT-4 | [HT-1] | H-1 | D-1 | Blue | Nil | Liq | E-2 | 4.8 | 3.9 | 125 |
| Comparative Example 32 | | | [HT-2] | | | | | | | 4.5 | 4.3 | 69 |
| Comparative Example 33 | | | [HT-3] | | | | | | | 4.5 | 4.3 | 89 |

**[Table 5-2]**

| | Hole injection layer | | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|---|---|
| | Acceptor compound | Hole injection material | | Host material | Dopant material | Emitted light color | | Donor compound | Electron transport material | | | |
| Comparative Example 34 | HAT-CN6 | Nil | [HT-1] | H-1 | D-1 | Blue | Nil | Liq | E-3 | 4.3 | 4.3 | 92 |
| Comparative Example 35 | | | [HT-2] | | | | | | | 4.0 | 4.6 | 51 |
| Comparative Example 36 | | | [HT-3] | | | | | | | 4.0 | 4.6 | 68 |
| Comparative Example 37 | HAT-CN6 | Nil | [HT-1] | H-1 | D-1 | Blue | Nil | Liq | E-4 | 4.3 | 4.3 | 95 |
| Comparative Example 38 | | | [HT-2] | | | | | | | 4.0 | 4.6 | 54 |
| Comparative Example 39 | | | [HT-3] | | | | | | | 4.0 | 4.6 | 74 |
| Comparative Example 40 | HAT-CN6 | Nil | [HT-1] | H-1 | D-1 | Blue | E-2 | Cesium | E-2 | 4.6 | 3.7 | 132 |
| Comparative Example 41 | | | [HT-2] | | | | | | | 4.2 | 4.1 | 70 |
| Comparative Example 42 | | | [HT-3] | | | | | | | 4.3 | 4.1 | 91 |
| Comparative Example 43 | F4-TCNQ | HT-4 | [HT-1] | H-1 | D-1 | Blue | E-2 | Cesium | E-2 | 4.7 | 3.8 | 125 |
| Comparative Example 44 | | | [HT-2] | | | | | | | 4.4 | 4.2 | 72 |
| Comparative Example 45 | | | [HT-3] | | | | | | | 4.3 | 4.2 | 93 |

**[Table 6]**

| | Hole injection layer | | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|---|---|
| | Acceptor compound | Hole injection material | | Host material | Dopant material | Emitted light color | | Donor compound | Electron transport material | | | |
| Comparative Example 46 | HAT-CN6 | Nil | [HT-1] | H-2 | D-2 | Green | Nil | Liq | E-1 | 12.1 | 3.9 | 187 |
| Comparative Example 47 | | | [HT-2] | | | | | | | 14.9 | 4.2 | 116 |
| Comparative Example 48 | | | [HT-3] | | | | | | | 15.2 | 4.2 | 134 |
| Comparative Example 49 | HAT-CN6 | Nil | [HT-1] | H-2 | D-2 | Green | Nil | Liq | E-2 | 11.9 | 3.7 | 172 |
| Comparative Example 50 | | | [HT-2] | | | | | | | 15.0 | 4.1 | 102 |
| Comparative Example 51 | | | [HT-3] | | | | | | | 15.0 | 4.1 | 126 |
| Comparative Example 52 | HAT-CN6 | Nil | [HT-1] | H-2 | D-2 | Green | Nil | Liq | E-3 | 12.2 | 4.1 | 134 |
| Comparative Example 53 | | | [HT-2] | | | | | | | 15.1 | 4.5 | 84 |
| Comparative Example 54 | | | [HT-3] | | | | | | | 15.4 | 4.4 | 112 |
| Comparative Example 55 | F4-TCNQ | [1] | [HT-1] | H-2 | D-2 | Green | Nil | Liq | E-1 | 12.1 | 3.8 | 180 |
| Comparative Example 56 | | | [HT-2] | | | | | | | 15.2 | 4.2 | 125 |
| Comparative Example 57 | | | [HT-3] | | | | | | | 14.9 | 4.2 | 136 |

**[Table 7]**

| | Hole injection layer | | Hole transport layer | Light-emitting layer | | | First electron transport layer | Second electron transport layer | | External quantum efficiency (%) at 10 mA/cm² | Drive voltage (V) at 10 mA/cm² | Durability life (hours) when luminance decreased by 20% |
|---|---|---|---|---|---|---|---|---|---|---|---|---|
| | Acceptor compound | Hole injection material | | Host material | Dopant material | Emitted light color | | Donor compound | Electron transport material | | | |
| Example 52 | HAT-CN6 | Nil | [2] | H-3 | D-3 | Red | E-5 | Cesium | E-5 | 10.2 | 5.0 | 620 |
| Example 53 | A-1 | HT-4 | [2] | H-3 | D-3 | Red | E-5 | Cesium | E-5 | 10.6 | 4.6 | 590 |
| Example 54 | HAT-CN6 | Nil | [2] | H-3 | D-3 | Red | E-6 | Cesium carbonate | E-6 | 11.8 | 4.8 | 680 |
| Example 55 | A-1 | HT-4 | [2] | H-3 | D-3 | Red | E-6 | Cesium carbonate | E-6 | 11.3 | 4.5 | 630 |
| Example 58 | HAT-CN6 | Nil | HT-2 | H-3 | D-3 | Red | E-5 | Cesium | E-5 | 9.3 | 5.2 | 340 |
| Example 59 | A-1 | HT-4 | HT-2 | H-3 | D-3 | Red | E-5 | Cesium | E-5 | 9.8 | 5.0 | 270 |
| Example 60 | HAT-CN6 | Nil | HT-2 | H-3 | D-3 | Red | E-6 | Cesium carbonate | E-6 | 10.1 | 5.0 | 355 |
| Example 61 | A-1 | HT-4 | HT-2 | H-3 | D-3 | Red | E-6 | Cesium carbonate | E-6 | 9.9 | 4.9 | 320 |

### Meanings of symbols

1 Energy level of an anode (work function)
2 Energy level of the HOMO level of a hole transport material
3 Energy level of the HOMO level of a light-emitting layer
4 Energy level of a cathode (work function)
5 Energy level of the LUMO level of the light-emitting layer
6 Energy level of the LUMO level of the hole transport material
7 Line showing the interface between the anode and a hall transport layer
8 Line showing the interface between the hole transport layer and a light-emitting layer
9 Line showing the interface between the light-emitting layer and the cathode
11 Hole on the anode
12 Hole injected into the hole transport material
13 Hole injected into the light-emitting layer
14 Electron on the cathode
15 Electron injected into the light-emitting layer
16 Line showing the recombination between the electron and the hole
17 Energy difference between LUMO level of the light-emitting layer and LUMO level of the hole transport layer

## Claims

1. A light-emitting element for emitting light by means of electric energy, in which at least a hole transport layer and an electron transport layer exist between an anode and a cathode, wherein the hole transport layer of the light-emitting element contains a compound represented by the following general formula (1), while the electron transport layer contains a donor compound, the donor compound being an alkali metal, an inorganic salt containing an alkali metal, a complex comprising an alkali metal and an organic substance, an alkaline earth metal, an inorganic salt containing an alkaline earth metal or a complex comprising an alkaline earth metal and an organic substance. 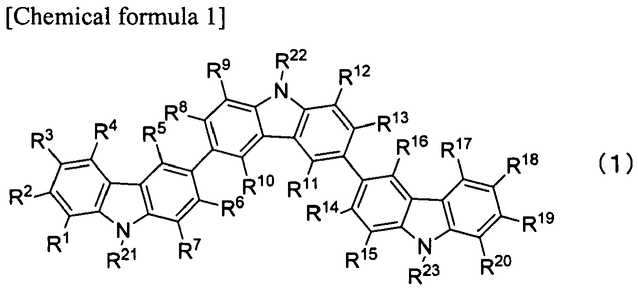 (R¹ to R²⁰ each are selected from the group comprising hydrogen, deuterium, alkyl group, cycloalkyl group, amino group, aryl group, heterocyclic group, heteroaryl group, alkenyl group, cycloalkenyl group, alkynyl group, alkoxy group, alkylthio group, aryl ether group, aryl thioether group, halogen, cyano group, -P(=O)R²⁴R²⁵ and silyl group; R²⁴ and R²⁵ each denote an aryl group or heteroaryl group; these substituent groups may also be further substituted, or substituent groups adjacent to each other may also form a ring; R²¹ to R²³ may be identical to or different from each other, and are selected from the group comprising an alkyl group, cycloalkyl group aryl group and heteroary group; and these substituent groups may also be further substituted.)

2. A light-emitting element, according to claim 1, wherein a hole injection layer exists between the hole transport layer and the anode and consists of an acceptor compound alone or contains an acceptor compound

3. A light-emitting element, according to claim 2, wherein the acceptor compound is a metal oxide or a cyano group-containing compound.

4. A light-emitting element, according to any one of claims 1 through 3, wherein the electron transport layer contains a phenanthroline derivative.

5. A light-emitting element, according to any one of claims 1 through 3, wherein the electron transport layer contains a compound having a pyrene skeleton or anthracene skeleton.

6. A light-emitting element, according to claim 5, wherein the compound having a pyrene skeleton or anthracene skeleton has a heteroaryl ring structure containing an electron-acceptable nitrogen.
